(19) **Europäisches Patentamt / European Patent Office / Office européen des brevets**

(11) **EP 4 663 187 A1**

(12) **EUROPEAN PATENT APPLICATION**
published in accordance with Art. 153(4) EPC

(43) Date of publication:
**17.12.2025 Bulletin 2025/51**

(21) Application number: **24770073.5**

(22) Date of filing: **13.03.2024**

(51) International Patent Classification (IPC):
**A61K 31/201** (2006.01)   **A61P 35/00** (2006.01)
**A61P 25/04** (2006.01)

(52) Cooperative Patent Classification (CPC):
**A61K 31/201; A61P 25/04; A61P 35/00**

(86) International application number:
**PCT/ES2024/070157**

(87) International publication number:
**WO 2024/189252 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.03.2023 ES 202330212**

(71) Applicant: **Laminar Pharmaceuticals, S.A.**
**07121 Palma de Mallorca (ES)**

(72) Inventors:
- **ESCRIBÁ RUIZ, Pablo Vicente**
  **07004 Palma de Mallorca (ES)**
- **LLADÓ CAÑELLAS, Victoria**
  **07011 Palma de Mallorca (ES)**
- **FERNÁNDEZ GARCÍA, Paula**
  **07007 Palma de Mallorca (ES)**

- **GERALD MCNICHOLL, Adrian**
  **07012 Palma de Mallorca (ES)**
- **ROSELLÓ CASTILLO, Catalina Ana**
  **07141 Marratxi (ES)**
- **FERRÁ CANET, Jordi**
  **07012 Palma de Mallorca (ES)**
- **TORRES CANALEJO, Manuel**
  **07014 Marratxi (ES)**
- **MIRALLES PIZÀ, Marc**
  **07120 Palma de Mallorca (ES)**
- **BETETA GOBEL, Roberto**
  **07160 Peguera (ES)**
- **JAUME BAZA, Catalina**
  **07240 Sant Joan (ES)**
- **CANO URREGO, Emilce**
  **1104 Bogotá (CO)**

(74) Representative: **Manuel Illescas y Asociados, S.L.U.**
**C/ Príncipe de Vergara 197, Oficina 1°A**
**28002 Madrid (ES)**

(54) **2-HYDROXY-OCTADECENE-9-CIS-OATE FOR USE IN THE TREATMENT OF ONCOLOGICAL PATHOLOGIES AND NEUROPATHIC PAIN**

(57) The present invention relates to the compound 2-hydroxy-octadecene-9-cis-oate for use in the treatment of oncological pathologies including grade IV glioblastoma with native IDH, grade 3 glioma, embryonal tumour, pituitary tumour, meningioma, haemangiopericytoma, haemangioblastoma, mesothelioma, bile duct adenocarcinoma, exocrine pancreatic cancer, neuroendocrine pancreatic cancer, rectal cancer, urachal cancer, adenocarcinoma of the bladder and oesophageal cancer, and for the treatment of neuropathic pain caused by diabetic peripheral neuropathy, spinal cord injury, postherpetic neuralgia, chemotherapeutic agent, antitumour agent, cancer, fibromyalgia or hepatitis. The present invention also relates to the pharmaceutical composition comprising 2-hydroxy-octadecene-9-cis-oate.

**Description**

**TECHNICAL FIELD OF THE INVENTION**

The present invention relates to the use of 2-hydroxy-octadecene-9-cis-oate (HOCO), a derivative of monounsaturated fatty acid hydroxylated in the alpha carbon, its salts or esters, for the treatment of oncological pathologies of the central nervous system and related pathologies and for the treatment of neuropathic pain caused by said oncological pathologies, due to their treatment with antitumour agents, chemotherapeutic agents, as well as peripheral diabetic neuropathy, postherpetic neuralgia, neuropathic pain after nerve or spinal injury, due to fibromyalgia or hepatitis.

**BACKGROUND TO THE INVENTION**

Lipids that are ingested in the diet, including fatty acids, can regulate the lipid composition of cell membranes. It is known that changes in the lipid composition of the membranes influence cell signalling, which can lead to the development of diseases, or reverse them, as well as preventing them. Similarly, therapeutic, nutraceutical or pharmacological interventions focused on regulating membrane lipid levels can prevent and reverse (cure) pathological processes.

At present, it is known that cell membranes are related to multiple cellular processes. On the one hand, they support proteins involved in cell signalling that regulates important organic parameters. This signalling, mediated by numerous hormones, neurotransmitters, cytokines, growth factors, etc., activates membrane proteins (receptors), which propagate the signal received into the cell through other proteins (peripheral membrane proteins), some of which are also located in the membrane. Since (1) these systems function as amplification cascades and (2) membrane lipids can regulate the localization and function of such peripheral proteins (plasma membrane or inner membrane or active or inactive cytoplasm), the lipid composition of the membranes can have a major impact on cell functionality. In particular, the interaction of certain peripheral proteins, such as G proteins, protein kinase C, RAS protein, etc., with the cell membrane depends on the lipid composition thereof. On the other hand, the lipid composition of cell membranes is influenced by the type and amount of lipids ingested. It follows that lipid intake can regulate the lipid composition of the membranes, which in turn can control the interaction (and therefore the activity) of important cell signalling proteins.

In the state of the art, studies have been carried out showing the therapeutic activity of 2-hydroxy-octadecene-9-cis-oate (HOCO), a derivative of monounsaturated fatty acid hydroxylated on the alpha carbon, in the treatment of various diseases.

There are patients suffering from various types of cancer with a poor prognosis with unmet medical needs. Likewise, there are patients suffering from neuropathic pain in which treatment with conventional analgesics is not sufficiently effective and who therefore have unmet medical needs. In view of this, there is a need to provide new treatments for these patients.

**DESCRIPTION OF THE INVENTION**

Thus, the term "pharmaceutically acceptable salt" refers to a salt or ester of a compound that also possesses the desired pharmacological activity of the parent compound from which it is derived.

The term "maintenance treatment" or "maintenance therapy" is defined as a therapeutic treatment administered as a complement to a main or primary treatment or therapy, for the purpose of either preventing or delaying the recurrence of the disease, which has completely or partially remitted after treatment with a primary treatment or therapy, or to slow the development of a disease after the end of treatment with a primary therapy.

The term "effective amount" or "therapeutically effective amount" refers to the amount of a drug that provides a therapeutic effect without providing unacceptable toxic effects on the patient. The effective amount or dose of the drug depends on the compound and the condition or disease treated, and for example the age, weight and clinical condition of the treated patient, the form of administration, the clinical history of the patient, the seriousness of the disease and the potency of the compound administered.

The term "RANO" (Response Assessment in Neuro-Oncology) refers to the criteria for evaluating the response in neuro-oncology, which are used in clinical trials and in clinical practice (Brandsma and van den Bent).

The term "RECIST" (Response Evaluation Criteria in Solid Tumors) refers to the evaluation criteria in solid tumours, which are used in patients with primary and/or metastatic cancer or recurrent metastatic disease (Lencioni et al.).

The term "PR" (Partial Response) refers to the partial response and corresponds to the decrease in the size of a tumour or in the degree of cancer in the body, in response to treatment. Also called partial remission.

The term "SD" (Stable Disease) refers to a stable disease, when the cancer does not decrease or increase in severity.

The term "AE" (Adverse Event) refers to an adverse effect, which is understood as an unexpected medical problem that happens during treatment with a drug or other therapy. Side effects are mild, moderate or severe and may have causes other than the medicine or therapy being given. Also called an adverse event.

The term "NCI-CTCAE" (National Cancer Institute-Common Terminology Criteria for Adverse Events) refers to the common terminological criteria for adverse events of the National Cancer Institute of the United States.

The problem of the state of the art consists in providing a compound for use in the treatment of several types of cancer with poor prognosis and/or short life expectancy and in the treatment of neuropathic pain, caused by peripheral diabetic neuropathy, post-herpetic neuralgia, spinal cord injury, chemotherapeutic agent, anti-tumour agent, cancer, fibromyalgia or hepatitis.

The present invention provides a solution to said technical problem.

The present invention provides the compound 2-hydroxy-octadecene-9-cis-oate, or a pharmaceutically acceptable salt or ester thereof, for use in the treatment of an oncological pathology selected from the group consisting of glioblastoma, astrocytoma, pilocytic astrocytoma, diffuse astrocytoma, anaplastic astrocytoma, oligoastrocytoma, oligodengroglioma, ependymoma, xanthastrocytoma, medulloblastoma, low-grade glioma, grade 3 glioma, pontine glioma, ependymal tumour, subependymoma, gliomatosis cerebri, embryonic tumour, atypical rhabdoid teratoid tumour, tumour of cranial and spinal nerves, mixed neuro-glial tumour, pituitary tumour, germ cell tumour, meningeal tumour, hemangiopericytoma, hemangioblastoma, tumour of the choroid plexus, papilloma of the choroid plexus, pineocytoma, pineoblastoma, mesothelioma, pleural mesothelioma, adenocarcinoma of the bile duct, exocrine pancreatic cancer, cancer of the neuroendocrine pancreas, metastatic adenocarcinoma of the lung, small cell lung cancer, adenocarcinoma of the colon, rectal cancer, cancer of the recto-sigmoid junction, rectal adenocarcinoma, metastatic rectal adenocarcinoma of the colon, colorectal adenocarcinoma, metastatic colorectal adenocarcinoma, urachal fistula cancer, urachal adenocarcinoma, adenocarcinoma of the endometrium, cancer of the neuroendocrine pancreas, adenocarcinoma of the pancreas, chondrosarcoma of the uterus, adenocarcinoma of the intestine cecum, bladder adenocarcinoma, esophageal cancer and metastatic small-cell esophageal cancer, which is administered at a dose of 500 mg/day to 16000 mg/day.

2-hydroxy-octadecene-9-cis-oate (HOCO) is a monounsaturated fatty acid of the formula COOH-$CHOH-(CH_2)_6-CH=CH-(CH_2)_7-CH_3$

The present invention also provides the use of the compound 2-hydroxy-octadecene-9-cis-oate, or a pharmaceutically acceptable salt or ester thereof, for the manufacture of a medicament for the treatment of an oncological pathology selected from the group consisting of glioblastoma, astrocytoma, pilocytic astrocytoma, diffuse astrocytoma, anaplastic astrocytoma, oligoastrocytoma, oligodengroglioma, ependymoma, xanthastrocytoma, medulloblastoma, low-grade glioma, grade 3 glioma, pontine glioma, ependymal tumour, subependymoma, cerebri gliomatosis, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, choroid plexus tumour, choroid plexus papilloma, pineocytoma, pineoblastoma, mesothelioma, pleural mesothelioma, bile duct adenocarcinoma, exocrine pancreatic cancer, neuroendocrine pancreatic cancer, metastatic adenocarcinoma of the lung, small cell lung cancer, colon adenocarcinoma, rectal cancer, recto-sigmoid junction cancer, rectal adenocarcinoma, metastatic rectal adenocarcinoma, metastatic sigmoid adenocarcinoma of the colon, colorectal adenocarcinoma, metastatic colorectal adenocarcinoma, urachal fistula cancer, urachal adenocarcinoma, endometrial adenocarcinoma, neuroendocrine pancreatic cancer, pancreas, chondrosarcoma of the uterus, adenocarcinoma of the caecum, adenocarcinoma of the bladder, esophageal cancer and metastatic small cell esophageal cancer, wherein said compound, salt or ester is administered in a dose of 500 mg/day to 16000 mg/day.

The present invention further provides a method of treating an oncological pathology selected from the group consisting of glioblastoma, astrocytoma, pilocytic astrocytoma, diffuse astrocytoma, anaplastic astrocytoma, oligoastrocytoma, oligodengroglioma, ependymoma, xanthoastrocytoma, medulloblastoma, low-grade glioma, grade 3 glioma, pontine glioma, ependymal tumour, subependymoma, cerebral gliomatosis, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, tumour of the choroid plexus, papilloma of the choroid plexus, pineocytoma, pineoblastoma, mesothelioma, pleural mesothelioma, bile duct adenocarcinoma, exocrine pancreatic cancer, neuroendocrine pancreatic cancer, metastatic lung adenocarcinoma, small cell lung cancer, colon adenocarcinoma, rectal cancer, rectal-sigmoid junction cancer, rectal adenocarcinoma, metastatic rectal adenocarcinoma, metastatic sigmoid adenocarcinoma of the colon, colorectal adenocarcinoma, metastatic colorectal adenocarcinoma, urachal fistula cancer, urachal adenocarcinoma, endometrial adenocarcinoma, neuroendocrine pancreatic cancer, pancreatic adenocarcinoma, uterine chondrosarcoma, blind bowel adenocarcinoma, bladder adenocarcinoma, esophageal cancer and metastatic microcytic esophageal cancer, comprising administering the compound 2-hydroxy-octadecene-9-cis-oate, or a pharmaceutically acceptable salt or ester thereof, to a subject at a dose of 500 mg/day to 16000 mg/day.

In phase I clinical studies with the sodium salt of HOCO (HOCOS, $COONa-CHOH-(CH_2)_6-CH=CH-(CH_2)_7-CH_3$) in monotherapy of patients with various types of cancer that did not respond to antitumour therapy, safety and efficacy of the compound against these types of cancer were observed (Example 1).

Brain tumours have different molecular and cellular characteristics, so their treatment varies according to the type of tumour. Moreover, it has been proven that paediatric brain tumours differ from tumours in adults, mainly from a molecular point of view, which means that their treatment is different from that applied to adult patients. When the brain tumour comes from glial cells, it is called glioma and is classified, according to the type of glial cell involved, into astrocytomas, ependymomas and oligodendrogliomas. Astrocytomas come from the connective cells called astrocytes. They are the most common intra-axial primary tumours. It is the most common type of glioma. Ependymomas develop from the ependymal cells that line the ventricles. They account for 2% of all brain tumours. They are the most common in children. Oligodendrogliomas are formed from oligodendrocytes, the brain's supporting cells. They represent between 1% and 2% of all brain tumours. They are more common in adults.

Glioblastoma is an astrocytoma that develops in the brain, cerebellum, or spinal cord. Glioblastomas account for about 15% of all brain tumours. Therefore, they are one of the most common types of primary brain tumours. Glioblastomas can affect people of any age, but they are more common in adults. Glioblastomas are very aggressive, grow quickly and can spread throughout the brain. They rarely spread outside the brain. Because of these characteristics, glioblastoma can be very difficult to treat and usually cannot be cured. In this sense, the average overall survival (OS) of patients with glioblastoma is about 14 months with the current standard treatment, which consists of radiation and temozolomide, a treatment that has not changed in the last 20 years.

At the time of conducting the human studies completed in the present invention, glioblastomas were considered to be high-grade gliomas (III and IV), defined by genetic and epigenetic alterations. Grade IV glioblastomas can have several mutations. The type of grade IV glioblastoma with the native isocitrate dehydrogenase (IDH) enzyme is the most aggressive and there are currently no effective treatments against this subtype of glioblastoma.

In embodiments of the compound, salt or ester for use of the invention, the oncological pathology is selected from the group consisting of oligoastrocytoma, glioblastoma, oligodengroglioma, ependymoma, xanthoastrocytoma, medulloblastoma, pilocytic astrocytoma, pontine glioma, ependymal tumor, subependymoma, gliomatosis cerebri, embryonal tumor, atypical teratoid rhabdoid tumor, cranial and spinal nerve tumor, mixed neuro-glial tumor, pituitary tumor, germ cell tumor, meninges tumor, meningioma, hemangiopericytoma, hemangioblastoma, choroid plexus tumor, choroid plexus papilloma, pineocytoma, pineoblastoma, mesothelioma, pleural mesothelioma, bile duct adenocarcinoma, exocrine pancreatic cancer, neuroendocrine pancreatic cancer, Metastatic lung adenocarcinoma, small cell lung cancer, rectal cancer, rectosigmoid junction cancer, metastatic rectal adenocarcinoma, metastatic sigmoid colon adenocarcinoma, metastatic colorectal adenocarcinoma, urachal fistula cancer, urachal adenocarcinoma, endometrial cancer, neuroendocrine pancreatic cancer, uterine chondrosarcoma, cecum adenocarcinoma, bladder adenocarcinoma, esophageal cancer, and metastatic small cell esophageal cancer.

In embodiments of the compound, salt or ester for use of the invention, the glioblastoma is grade IV glioblastoma multiforme with the native isocitrate dehydrogenase (IDH) enzyme.

In embodiments, the compound, salt or ester for use of the invention is for use in the treatment of native IDH grade IV glioblastoma multiforme in a subject having methylation of the promoter of the methyl guanine methyl transferase (MGMT) gene.

In embodiments of the compound, salt or ester for use of the invention, the salt is sodium salt.

In embodiments of the compound, salt or ester for use of the invention, the ester is methyl ester or ethyl ester.

In embodiments of the invention, the compound, salt or ester is administered orally.

In embodiments of the invention, the compound, salt or ester is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day 8000 mg/day, 10000 mg/day, 12000 mg/day, 12600 mg/day, 14000 mg/day and 16000 mg/day. Preferably, the compound, salt or ester of the invention is administered in a dose of 2100 mg/day or 12000 mg/day.

In embodiments of the invention, the compound, salt or ester is administered in two doses per day, in doses selected from the group consisting of 250 mg, 500 mg, 1000 mg, 2000 mg, 4000 mg, 6000 mg and 8000 mg.

In embodiments of the invention, the compound, salt or ester is administered in three doses per day, in doses selected from the group consisting of 1050 mg, 2100 mg, 4000 mg and 4200 mg.

In embodiments of the invention, the compound, salt or ester is administered in a dose of 2100 mg/day in three doses per day of 700 mg each.

In embodiments of the invention, the compound, salt or ester is administered in a dose of 12000 mg/day in three doses per day of 400 mg each.

In embodiments of the invention, the compound, salt or ester is used as a first line treatment.

In embodiments of the compound, salt or ester for use of the invention, the first line treatment comprises 4-week cycles, wherein each cycle comprises administering the compound, salt or ester daily during the first three weeks of each cycle and wherein during the fourth week no treatment is administered.

In embodiments of the invention, the compound, salt or ester is used as a pre-surgical or post-surgical treatment to decrease the size of a tumour.

In embodiments of the compound, salt or ester for use of the invention, the subject receiving the treatment has been previously treated with at least one and up to five different lines of chemotherapeutic treatment.

In embodiments of the invention, the compound, salt or ester is used as a maintenance treatment.

In embodiments of the compound, salt or ester for use of the invention, the maintenance treatment comprises 4-week cycles, wherein each cycle comprises administering the compound, salt or ester daily during the first three weeks of each cycle and wherein during the fourth week no treatment is administered.

In a phase II/III clinical trial which forms part of the present invention, first-line treatment of HOCOS combined with radiotherapy and temozolomide was investigated in newly diagnosed patients with native trial grade IV glioblastoma multiforme (Example 2). The treatment of various types of cancer with HOCOS, in combination with various chemotherapeutic agents, was investigated in cell line trials (Example 3).

In embodiments of the compound, salt or ester for use of the invention, the treatment comprises:

- a chemoradiotherapy phase lasting from 6 to 7 weeks, comprising administering radiotherapy daily, 5 days per week; administering a second chemotherapeutic agent in a daily dose of 75 mg/m$^2$ from the first day of radiotherapy; and administering the compound, salt or ester in a dose of 12000 mg/day for 4 weeks from week 3 counted from the start of radiotherapy;
- a 4-week treatment rest period;
- a 4-week, 6-cycle maintenance period, wherein each cycle comprises administering the second chemotherapeutic agent at a daily dose of 150-200 mg/m$^2$, the first five days of each cycle; administering the compound, salt or ester at a dose of 12000 mg/day for the first three weeks of each cycle and wherein during the fourth week no treatment is administered; and
- a monotherapy phase of 4-week cycles, wherein each cycle comprises administering the compound, salt or ester in a dose of 12000 mg/day during the first three weeks of each cycle, wherein during the fourth week no treatment is administered, wherein said cycles continue indefinitely as maintenance therapy.

In the previous embodiment and in other subsequent embodiments, certain daily doses have been expressed in mg/m$^2$, based on the body surface of the subject to be treated, as is usual in the state of the art.

In preferred embodiments of the compound, salt or ester for use of the invention, the treatment comprises:

- a chemoradiotherapy phase lasting from 6 to 7 weeks, comprising administering radiotherapy daily, 5 days per week; administering temozolomide in a daily dose of 75 mg/m$^2$ from the first day of radiotherapy; and administering HOCOS in a dose of 12000 mg/day for 4 weeks from week 3 counted from the start of radiotherapy;
- a 4-week treatment rest period;
- a 4-week 6-cycle maintenance period, wherein each cycle comprises administering temozolomide at a daily dose of 150-200 mg/m$^2$, on the first five days of each cycle; administering HOCOS at a dose of 12000 mg/day for the first three weeks of each cycle and wherein during the fourth week no treatment is administered; and
- a monotherapy phase of 4-week cycles, wherein each cycle comprises administering the compound, salt or ester in a dose of 12000 mg/day during the first three weeks of each cycle, wherein during the fourth week no treatment is administered, wherein said cycles continue indefinitely as maintenance therapy.

In embodiments of the compound, salt or ester for use of the invention, the radiotherapy is administered in a daily dose of 2 Gy.

In embodiments, the compound, salt, or ester of the invention is for simultaneous, separate, or sequential use in combination with a second chemotherapeutic agent selected from the group consisting of temozolomide, thiamine, gemcitabine, fluorouracil, oxyplatin, irinotecan, etoposide, imatinib, folfirinox, erlotinib, and cisplatin.

In embodiments, the compound, salt or ester for use of the invention is for simultaneous, separate or sequential use in combination with temozolomide, in the treatment of glioblastoma. Preferably, the glioblastoma is native IDH grade IV glioblastoma multiforme.

The invention also provides the compound 2-hydroxy-octadecene-9-cis-oate, or a pharmaceutically acceptable salt or ester thereof, for use in the treatment of neuropathic pain, which is administered at a dose of 500 mg/day to 16000 mg/day.

Neuropathic pain is not a conventional pain triggered by specific analgesic pain receptors, characteristic of cuts, bumps, burns and the like. Neuropathic pain may originate from various types of events including neuronal injury, central and/or peripheral nervous system nerve injury, a chemotherapeutic agent, an antitumour agent, cancer, diabetic peripheral neuropathy, postherpetic neuralgia, fibromyalgia, and hepatitis.

Most patients suffering from this type of neuropathic pain are treated with several very powerful and toxic drugs, with very modest therapeutic results. An example is spinal cord injury, which has been shown to be resistant to treatment with opioids, a family of drugs that are usually very potent for other types of pain (Rodgers et al.). In this way, patients with neuropathic pain receive anticonvulsants, anxiolytics, antidepressants, opioids and even anaesthetics, which allow them to only tolerate it momentarily.

The present invention also provides the use of the compound 2-hydroxy-octadecene-9-cis-oate, or a pharmaceutically acceptable salt or ester thereof, for the manufacture of a drug for the treatment of neuropathic pain.

The present invention further provides a method of treating neuropathic pain comprising administering an effective amount of the compound 2-hydroxy-octadecene-9-cis-oate, or a pharmaceutically acceptable salt or ester thereof, to a subject.

In embodiments of the compound, salt or ester for use of the invention, neuropathic pain is caused by a neuronal injury or by a nerve injury of the central and/or peripheral nervous system. Preferably, the neuronal injury is a spinal cord injury.

In embodiments of the compound, salt, or ester for use of the invention, the neuropathic pain is caused by a cause selected from the group consisting of a chemotherapeutic agent, an antitumour agent, and cancer. Preferably, the antitumour agent is an alkaloid. More preferably, the alkaloid is vincristine.

In embodiments of the compound, salt or ester for use of the invention, the neuropathic pain is caused by a cause selected from the group consisting of peripheral diabetic neuropathy, postherpetic neuralgia, spinal cord injury, chemotherapeutic agent, antitumour agent, cancer, fibromyalgia and hepatitis.

In embodiments, the compound, salt or ester for use of the invention in the treatment of neuropathic pain is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day, 8000 mg/day, 10000 mg/day, 12000 mg/day, 12600 mg/day, 14000 mg/day and 16000 mg/day. Preferably, the compound, salt or ester of the invention is administered in a dose of 2100 mg/day or 4200 mg/day.

In preferred embodiments of the compound, salt or ester for use of the invention, the neuropathic pain originates from a cause selected from the group consisting of diabetic peripheral neuropathy, postherpetic neuralgia, fibromyalgia and hepatitis.

In embodiments, the compound, salt, or ester of the invention is for simultaneous, separate, or sequential use in combination with at least one other active ingredient selected from the group consisting of pregabalin, an opioid analgesic, a steroidal analgesic, a non-steroidal analgesic, a cannabinoid analgesic, an anticonvulsant, an anxiolytic, an anaesthetic, and an antidepressant.

In embodiments, the compound, salt or ester of the invention is for simultaneous use, separately or sequentially in combination with at least one other active ingredient selected from the group consisting of lamotrigine, omeprazole, phenoxypentanoic acid, metformin, ibuprofen, dulcolax, alprazolam, diazepam, baclofen, macrogol, duloxetine, levofloxacin, gabapentin, ceftriaxone, enoxaparin, pantoprazole, paracetamol, ipratropium bromide, acetylcysteine, metoclopramide, metamizole, teicoplanin, dexketoprofen, oxybutynin, meropenem, beclomethasone dipropionate, formoterol fumarate, atorvastatin, calcifediol, paroxetine, cyanocobalamin, clonazepam, amlodipine, trazodone, flurazepam, thiazides, losartan, metamizole, sucralfate, lactitol, pancreatin, dimethicone, bethanechol, amitriptyline, lorazepam, vitamin D, lormetazepam, solifenacin, simvastatin, cinitapride, quetiapine, betmiga, valproate, lacosamide, lidocaine, metamizole, finasteride, acetylsalicylic acid, enalapril, metformin, liraglutide, trospium, tramadol, celcoxib, citalopram, sildenafil, tamsulosin, loratadine, tizanidine, vortioxetine, zolpidem, bromazepam, dexamethasone, dexketoprofen, losartan, eslicarbazepine, pentoxifylline, mirabegron, fluoxetine, clorazepate, mirtazapine, rosuvastatin, prednisone, beclamethasone, methylprednisone, acyclovir, lignocaine, nystatin, delorazepam, alendronic acid, carbamazepine, cortisone, hydrocortisone, cannabidiol and tetrahydrocannabinol.

The present invention also provides a pharmaceutical composition comprising the compound 2-hydroxy-octadecene-9-cis-oate, or a pharmaceutically acceptable salt or ester thereof, together with at least one pharmaceutically acceptable excipient or carrier, for use in the treatment of an oncological pathology, selected from the group consisting of glioblastoma, astrocytoma, pilocytic astrocytoma, diffuse astrocytoma, anaplastic astrocytoma, oligoastrocytoma, oligodengroglioma, ependymoma, xanthastrocytoma, medulloblastoma, low-grade glioma, grade 3 glioma, pontine glioma, ependymal tumour, subependymoma, gliomatosis cerebri, embryonic tumour, atypical rhabdoid teratoid tumour, cranial nerve tumour and spinal, mixed neuro-glial tumour, pituitary tumour, germ cell tumour, meninges tumour, meningioma, hemangiopericytoma, hemangioblastoma, tumour of the choroid plexus, papilloma of the choroid plexus, pineocytoma, pineoblastoma, mesothelioma, pleural mesothelioma, bile duct adenocarcinoma, exocrine pancreatic cancer, cancer of the neuroendocrine pancreas, metastatic adenocarcinoma of the lung, small cell lung cancer, colon adenocarcinoma, rectal cancer, cancer of the recto-sigmoid junction, rectal adenocarcinoma, metastatic rectal adenocarcinoma, metastatic sigmoid adenocarcinoma of the colon, colorectal adenocarcinoma, metastatic colorectal adenocarcinoma, urachal fistula cancer, urachal adenocarcinoma, endometrial adenocarcinoma, neuroendocrine pancreatic cancer, pancreatic adenocarcinoma, chondrosarcoma of the uterus, adenocarcinoma of the cecum, adenocarcinoma of the bladder, esophageal cancer and metastatic small cell esophageal cancer, wherein the compound, salt or ester is administered in a dose of 500 mg/day to 16000 mg/day.

In embodiments of the pharmaceutical composition of the invention, the oncological pathology is selected from the group consisting of oligoastrocytoma, glioblastoma, oligodengroglioma, ependymoma, xanthoastrocytoma, medulloblastoma, pilocytic astrocytoma, pontine glioma, ependymal tumor, subependymoma, gliomatosis cerebri, embryonal tumor, atypical teratoid rhabdoid tumor, tumor of cranial and spinal nerves, mixed neuro-glial tumor, pituitary tumor, germ cell tumor, meninges tumor, meningioma, hemangiopericytoma, hemangioblastoma, choroid plexus tumor, choroid plexus papilloma, pineocytoma, pineoblastoma, mesothelioma, pleural mesothelioma, bile duct adenocarcinoma, exocrine pancreatic cancer, neuroendocrine pancreatic cancer, metastatic adenocarcinoma of lung, small cell lung cancer, rectal cancer, rectosigmoid junction cancer, metastatic rectal adenocarcinoma, metastatic sigmoid colon adenocarcinoma, metastatic colorectal adenocarcinoma, urachal fistula cancer, urachal adenocarcinoma, endometrial cancer, neuroendocrine pancreatic cancer, uterine chondrosarcoma, cecum adenocarcinoma, bladder adenocarcinoma, esophageal cancer, and metastatic small cell esophageal cancer.

In embodiments of the pharmaceutical composition of the invention, the glioblastoma is grade IV glioblastoma multiforme with the native isocitrate dehydrogenase (IDH) enzyme.

In embodiments, the pharmaceutical composition of the invention is for use in the treatment of native trial grade IV glioblastoma multiforme in a subject having methylation of the promoter of the methyl guanine methyl transferase (Mgmt) gene.

In embodiments of the pharmaceutical composition of the invention, the salt is sodium salt (HOCOS).

In embodiments of the pharmaceutical composition of the invention, the ester is methyl ester or ethyl ester.

In embodiments of the pharmaceutical composition of the invention, the compound, salt or ester is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day, 8000 mg/day, 10000 mg/day, 12000 mg/day, 12600 mg/day, 14000 mg/day and 16000 mg/day. Preferably, the compound, salt or ester of the invention is administered in a dose of 2100 mg/day or 12000 mg/day.

In embodiments, the pharmaceutical composition of the invention additionally comprises a second chemotherapeutic agent selected from the group consisting of temozolomide, thiamine, gemcitabine, fluorouracil, oxyplatin, irinotecan, etoposide, imatinib, folfirinox, erlotinib, and cisplatin.

In embodiments, the pharmaceutical composition of the invention further comprises temozolomide, for use in the treatment of glioblastoma. Preferably, the glioblastoma is native IDH grade IV glioblastoma multiforme.

The present invention also provides a pharmaceutical composition comprising the compound 2-hydroxy-octadecene-9-cis-oate, or a pharmaceutically acceptable salt or ester thereof, together with at least one pharmaceutically acceptable excipient or carrier, for use in the treatment of neuropathic pain, wherein the compound, salt or ester is administered at a dose of 500 mg/day to 16000 mg/day.

In embodiments of the pharmaceutical composition of the invention, the neuropathic pain is caused by a neuronal injury or by a nerve injury of the central and/or peripheral nervous system. Preferably, the neuronal injury is a spinal cord injury.

In embodiments of the pharmaceutical composition of the invention, the neuropathic pain is caused by a cause selected from the group consisting of a chemotherapeutic agent, an antitumour agent and cancer. Preferably, the antitumour agent is an alkaloid. More preferably, the alkaloid is vincristine.

In embodiments of the pharmaceutical composition of the invention, the neuropathic pain is caused by a cause selected from the group consisting of diabetic peripheral neuropathy, postherpetic neuralgia, fibromyalgia and hepatitis.

In embodiments of the pharmaceutical composition of the invention for use in the treatment of neuropathic pain, the compound, salt or ester is administered in a dose selected from the group consisting of 500 mg/day, 1000 mg/day, 1050 mg/day, 2000 mg/day, 2100 mg/day, 4000 mg/day, 4200 mg/day, 6000 mg/day, 8000 mg/day, 10000 mg/day, 12000 mg/day, 14000 mg/day and 16000 mg/day. Preferably, the compound, salt or ester of the invention is administered in a dose of 2100 mg/day or 4200 mg/day.

In embodiments, the pharmaceutical composition of the invention additionally comprises at least one other active ingredient selected from the group consisting of pregabalin, an opioid analgesic, a steroidal analgesic, a non-steroidal analgesic, a cannabinoid analgesic, an anticonvulsant, an anxiolytic, an anaesthetic, and an antidepressant.

In embodiments, the pharmaceutical composition of the invention further comprises at least one other active ingredient selected from the group consisting of lamotrigine, omeprazole, phenoxypentanoic acid, metformin, ibuprofen, dulcolax, alprazolam, diazepam, baclofen, macrogol, duloxetine, levofloxacin, gabapentin, ceftriaxone, enoxaparin, pantoprazole, paracetamol, ipratropium bromide, acetylcysteine, metoclopramide, metamizole, teicoplanin, dexketoprofen, oxybutynin, meropenem, beclomethasone dipropionate, formoterol fumarate, atorvastatin, calcifediol, paroxetine, cyanocobalamin, clonazepam, amlodipine, trazodone, flurazepam, thiazides, losartan, metamizole, sucralfate, lactitol, pancreatin, dimethicone, bethanechol, amitriptyline, lorazepam, vitamin D, lormetazepam, solifenacin, simvastatin, cinitapride, quetiapine, betmiga, valproate, lacosamide, lidocaine, metamizole, finasteride, acetylsalicylic acid, enalapril, metformin, liraglutide, trospium, tramadol, celcoxib, citalopram, sildenafil, tamsulosin, loratadine, tizanidine, vortioxetine, zolpidem, bromazepam, dexamethasone, dexketoprofen, losartan, eslicarbazepine, pentoxifylline, mirabegron, fluoxetine, clorazepate, mirtazapine, rosuvastatin, prednisone, beclamethasone, methylprednisone, acyclovir, lignocaine, nystatin, delorazepam, alendronic acid, carbamazepine, cortisone, hydrocortisone, cannabidiol and tetrahydrocannabinol.

In embodiments, the pharmaceutical composition of the invention further comprises 1 to 20% (w/w) of an antioxidant, 0.01 to 10% (w/w) of a sweetener, 0.1 to 20% (w/w) of a gelling agent, and 0.01 to 10% (w/w) of a flavouring, the sum of the percentages of all ingredients of the pharmaceutical composition (including the active ingredient(s)) being 100% (w/w).

In embodiments, the pharmaceutical composition of the invention further comprises 20 to 80% (w/w) of a diluent, 0.1 to 20% (w/w) of an antioxidant, 0.01 to 10% (w/w) of a sweetener, 0.1 to 20% (w/w) of a gelling agent, and 0.01 to 10% (w/w) of a flavouring, the sum of the percentages of all ingredients of the pharmaceutical composition (including the active ingredient(s)) being 100% (w/w).

The pharmaceutical composition of the present invention may be in the form of a gastro-resistant compound to prevent degradation of its components by the low pH of the gastric environment. The pharmaceutically acceptable excipient or carrier includes, but is not limited to, diluents, antioxidants, sweeteners, gellants, flavourings, fillers, or other vehicles, such as colloidal anhydrous silica and glyceryl monostearate. The pharmaceutical composition can be in the form of a capsule, powders, tablets, emulsion, suspension, solution or any other pharmaceutical form, packaged in a blister, bottle, a paper, polyester, polyethylene and/or aluminium sachet, or any other type of container. To produce the pharmaceutical composition of the invention, conventional techniques for preparing pharmaceutical compositions can be used. For example, the compound, salt or ester of the invention, disclosed herein, may be mixed with a carrier, or diluted by a carrier, or enclosed within a carrier that may be in the form of an ampoule, capsule, envelope, paper, polyethelene and/or aluminium or other packaging. When the carrier is a diluent, it may be a solid, semi-solid, or liquid material that acts as a vehicle, excipient, or medium for the active compound. Some examples of suitable diluentss are water, saline solutions, alcohols, polyethylene glycols, polyhydroxyethoxylated castor oil, peanut oil, olive oil, lactose, terra alba, sucrose, cyclodextrins, amylose, magnesium stearate, talcum, gelatine, agar, pectin, acacia, stearic acid, cellulose alkyl ethers, silicic acid, fatty acids, fatty acid amines, fatty acid monoglycerides and diglycerides, fatty esters of pentaerythrol, polyethylene, hydroxymethylcellulose and polyvinylpirrolidone. Similarly, the carrier or diluent can include any sustained release material known in the art, such as glyceryl monostearate or glyceryl distearate, alone or mixed with a wax. The pharmaceutical composition of the invention may also include wetting agents, antioxidants, emulsifying and suspending agents, preserving agents, sweetening agents, and flavouring agents. The pharmaceutical composition of the invention may be formulated to provide rapid, sustained or delayed release of the compounds disclosed herein after administration to the patient using methods well known in the art.

The pharmaceutical composition of the invention may be a solid composition or a liquid solution.

The pharmaceutical composition of the invention may be sterilized and mixed, if desired, with auxiliary agents, emulsifiers, salt to influence osmotic pressure, buffers and/or colouring substances and the like, which do not react adversely with the compounds disclosed above.

Throughout the description and the claims, the term "comprising", "that comprises" and their variants are meant in a non-limiting sense and therefore should not exclude other technical features. The term "comprises", "comprising" and its variants, throughout the description and claims, specifically includes the term "consists of", "consisting of" and their variants.

As used herein and in the claims, the singular form "the" includes references to plural forms unless the content clearly indicates otherwise.

Unless defined otherwise, all the technical and scientific terms used throughout the description and claims have the same meaning as those customarily understood by a person skilled in the field of the invention.

**BRIEF DESCRIPTION OF THE FIGURES**

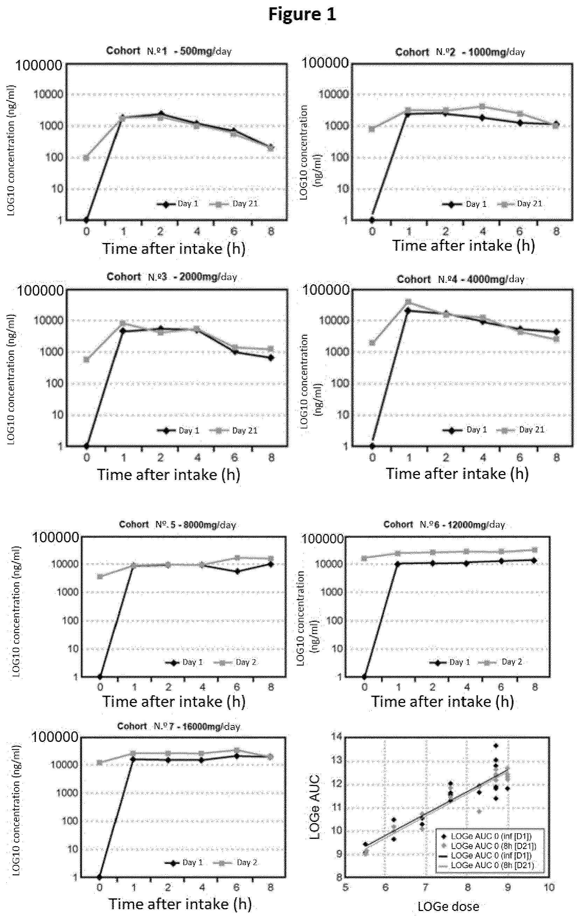

**Figure 1. Pharmacokinetic profile of HOCOS at different doses.** The graphs represent a serum log [HOCOS] (ng/ml) of patients after the administration of the first dose of the first day of treatment (day 1, black) and steady state, after 21 days of treatment (day 21, grey) at doses of 500 mg/day (b.i.d.), 1000 mg/day (b.i.d.), 2000 mg/day (b.i.d.), 4000 mg/day (b.i.d.), 8000 mg/day (b.i.d.), 12000 mg/day (t.i.d.) and 16000 mg/day (b.i.d.), orally. These daily doses were divided into two (b.i.d.) or three (t.i.d.) daily doses. These results demonstrate the good absorption of HOCOS at different doses in patients with different types of cancer (see Table 2).

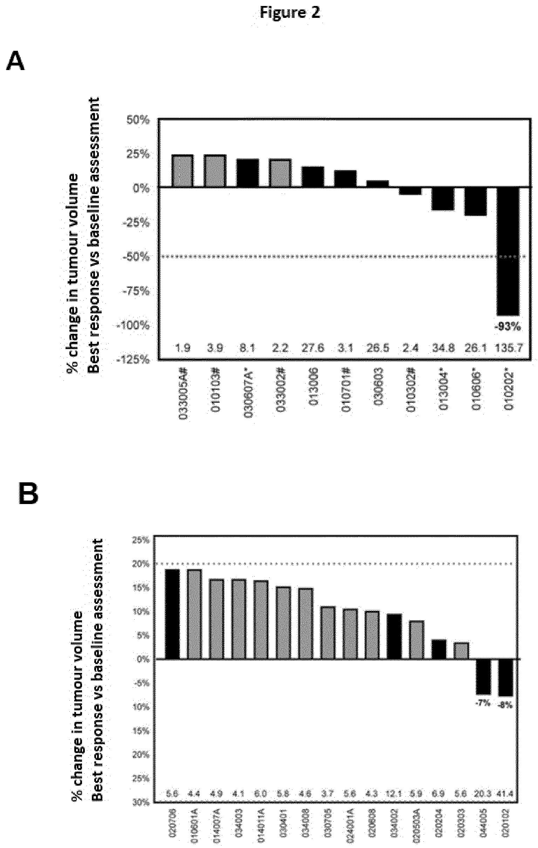

**Figure 2. Anti-tumour efficacy during HOCOS monotherapy.** In a phase I/II clinical trial, the safety and efficacy of HOCOS was studied in patients with different types of cancer (Table 2). In these patients (designated with a code) the volume of the tumour was determined at the end of the clinical trial (% volume with respect to the initial diagnosis). **(A)** tumour volume of glioblastoma patients relative to baseline volume at the time of recruitment (0%). The dotted lines describe the upper and lower limits to consider tumour progression, stable disease, and tumour regression according to the RANO criteria. **(B)** tumour volume in patients with non-glioblastoma tumours with respect to baseline volume (0%). In both graphs, the grey bars are patients treated with HOCOS and with stable disease or tumour regression that did not meet all RANO or RECIST criteria and the black bars are patients treated with HOCOS and with stable disease or tumour regression that did meet all RANO or RECIST criteria.

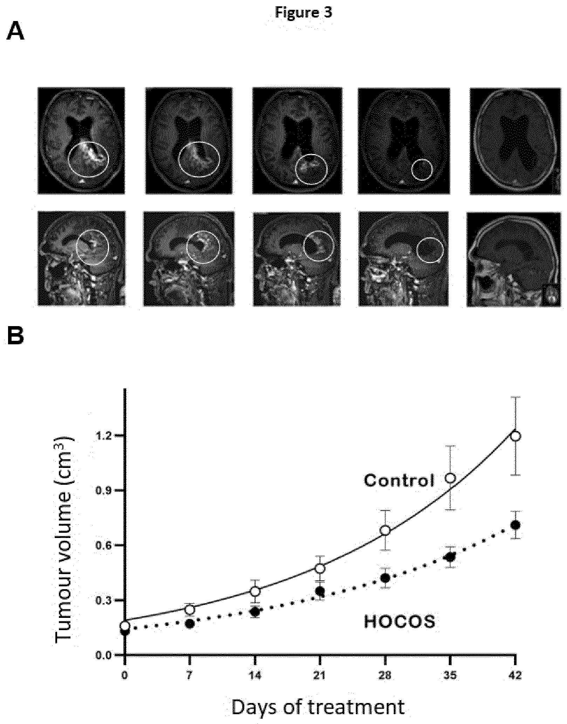

**Figure 3. Example of response to HOCOS treatment. (A)** Resonance imaging (MRI) of a glioblastoma patient, showing tumour size at baseline (first column images from the left) and progression during the time HOCOS was administered as the only therapy (phase I/II clinical trial), at 4 months (second column images from the left), 9 months (third column images from the left), 21 months (fourth column images from the left), and 32 months (fifth column images from the left). The tumour appears as the white area within the white circle in the middle of the normal neural tissue (grey). The patient showed sustained tumour regression over time (according to the RANO criteria) for three years in monotherapy with HOCOS 500 mg orally twice a day (1000 milligrams a day, p.o., b.i.d.). Transverse brain (upper scans) and sagittal (lower scans) MRI scans show the reduction of glioblastoma (within the white circle) throughout the HOCOS monotherapy treatment period. **(B)** Effect of HOCOS (black dots) on a murine (immunocompromised mice) model of human pancreatic cancer (MIAPaCa-2 cells). The image shows how the treatment slows the growth rate of pancreatic cancer compared to the control (white dots).

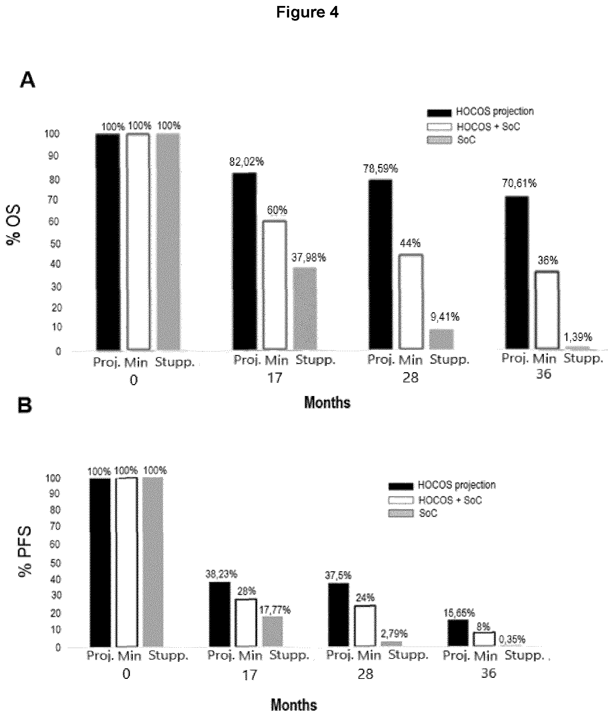

**Figure 4. Efficacy of HOCOS as standard first-line treatment in combination with temozolomide and radiotherapy in newly diagnosed patients with native trial grade IV glioblastoma.** In a phase II/III clinical trial, the safety and efficacy of HOCOS in combination with TMZ and other drugs was evaluated. **(A)** Total *survival of patients (OS),* which corresponds to the time between diagnosis and death of the same. Treatment with HOCOS (black bars) significantly increases OS. **(B)** Progression-free survival (PFS), which corresponds to the time (months) between diagnosis and the increase in tumour size according to RANO criteria. The grey bars show survival in patients treated with the current standard of care (SoC), consisting of radiotherapy for 6 weeks and temozolomide for 6 months, the white bars show survival in the clinical trial with the current standard of care plus HOCOS or placebo (approximately 50% of each treatment concomitant to temozolomide for 6 months and monotherapy thereafter) and the black bars are the extrapolation of the survival of patients treated with HOCOS plus the standard of care, considering that in the clinical trial the patients were randomized with a 1:1 ratio between the groups treated with HOCOS and placebo. In addition, patients were administered other drugs mentioned in the present invention during treatment.

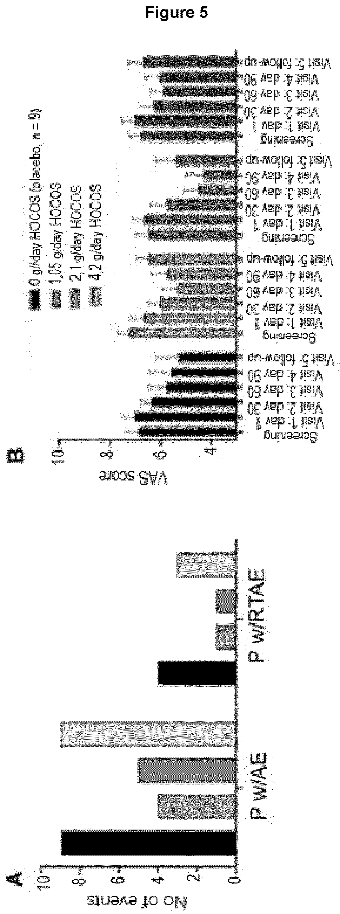

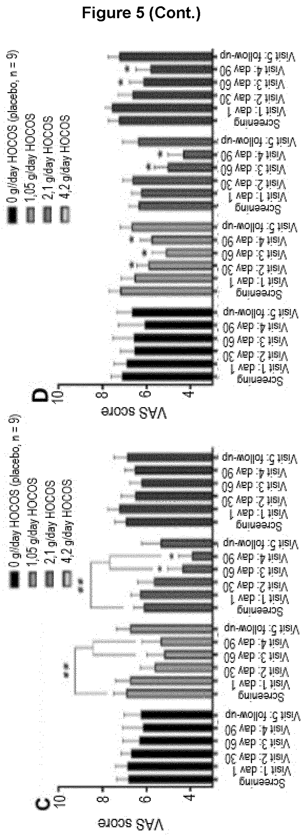

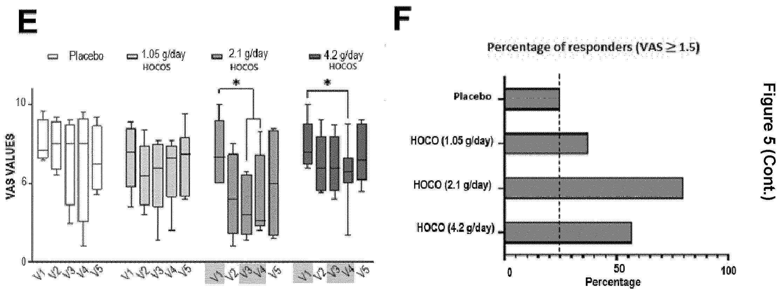

**Figure 5. Safety and efficacy of HOCOS in the treatment of neuropathic pain in patients with spinal cord injury. (A)** To evaluate the pharmacological safety of the product, it is studied whether an adverse effect (AE) is related to the treatment or not. The medical researchers determined whether or not the AEs were treatment-related. The phase I/II clinical trial in patients with spinal cord injury consisted of 4 groups to which patients were randomly assigned. The first group was given placebo (n=9), the second group was given 1050 mg of HOCOS divided into three intakes of 350 mg each (n=11), the third group was given 2100 mg of HOCOS divided into three intakes of 700 mg each (n=11), and the fourth group was given 4200 mg of HOCOS divided into three intakes of 1400 mg each. All patients also received pregabalin together with other drugs mentioned in the present invention. The bars on the left show all adverse effects in patients treated with placebo or different doses of HOCOS (P w/AE). The bars on the right show the adverse effects in patients that the clinical investigators identified as directly related to treatment (P w/RTAE) with HOCOS or with placebo. **(B)** In order to determine the efficacy of HOCOS versus placebo in patients with neuropathic pain caused by spinal cord injury, the VAS scale *(visual analogue scale)* was used. Pain in the total patient population (panel B) was determined during the screening visit, during the baseline visit (V1), at the end of the first month (V2), second month (V3) and third month of treatment (V4) and after one month without treatment (V5, *follow-up).* The reduction between baseline and end of trial was calculated in the total patient population as V1-V4. After the end of the trial, V5-V4 was analysed, which had to be positive in all cases, since when leaving the treatment an increase in pain is expected, either due to the drug or the placebo effect. Graph B shows pain values (VAS) in patients who received 0.0 (placebo), 1050, 2100 or 4200 milligrams daily of HOCOS. **(C)** Since the sensation of neuropathic pain can fluctuate over time, it can be reduced and even disappear naturally, a pain assessment was carried out at the end of the trial treatment period (V5). The trial lasted approximately 6 months between screening and V5 and it was observed that some patients showed a continuous trend in their pain reduction that continued after the end of treatment (between V4 and V5). It was expected that between V4 and V5 there would be an increase in the sensation of pain, even (although to a lesser extent) in the placebo group. This abnormal behaviour was eliminated in a second analysis, censoring those patients with pain reduction between V4 and V5 or with other abnormalities, such as erratic values. In this censored population, results very similar to those of the total population were obtained, but statistically significant (two-way ANOVA, Fisher's least significant difference test - Fisher's LSD). **(D)** Pain values on the VAS scale measured in responder patients, which are the population of uncensored patients who experienced a reduction in pain of more than 1 point on the pain scale (between V1 and V4) and who presented an increase in pain after completing treatment (between V4 and V5). The results show the values ± mean for each group and time point. A two-tailed ANOVA followed by a Fisher post-hoc LSD analysis was used to determine the significance of the data. *p < 0.05 with respect to baseline pain. **(E)** VAS values in patients with a probability of more than 90% of suffering from neuropathic pain, based on the "PainDetect" test, performed in all patients. The reduction in pain was statistically significant at the doses of 2100 mg/day and 4200 mg/day. The dose of 2100 mg/day was the most appropriate, showing reductions from the first month, being significant from the second and reaching the maximum by the third month of treatment, with a reduction from VAS values of about 7 to about 3. **(F)** Among the patient population with a greater than 90% probability of suffering from neuropathic pain, the percentage of responding patients (reduction in the VAS scale ≥ 1.5 points) in the placebo group (treated with pregabalin and other drugs, but not with HOCOS) was only 25%. In contrast, all groups of patients treated with HOCOS had a percentage of response to treatment greater than 40%, with the 2100 mg/day group again having the highest percentage of response, with 80%.

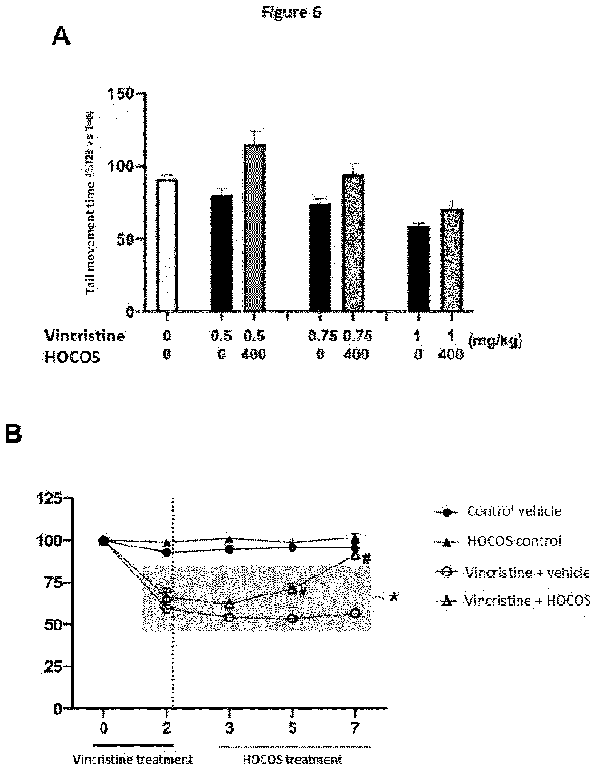

**Figure 6. Efficacy of HOCOS measured on thermal hyperalgesia by means of the "tail flick" test against vincristine-induced neuropathic pain. (A)** Animals were first treated with 0.0 (control), 0.5, 0.75 or 1 mg/kg vincristine (cumulative dose over 10 days). Vincristine is a drug used in cancer chemotherapy and associated with the onset of neuropathic pain. Then, the same animals were orally treated with saline (0 mg/kg HOCOS) or HOCOS (400 mg/kg) for 28 days. The latency time in the indicated animal groups was measured after 28 days of treatment. HOCOS was shown to increase latency time in the tail flick test, which is interpreted as a reduction in neuropathic pain. Values were expressed as a % response time to stimulus on day 28 of treatment versus baseline (day 1). **(B)** Effect of HOCOS on thermal hyperalgesia caused by vincristine (1 mg/kg) and measured by the tail flick test. Such hyperalgesia is a result of neuropathic pain, and reaction time to heat in animals on the first day and after 7 weeks of HOCOS treatment is shown. This shows that vincristine-induced reductions in the response time to heat discomfort, which could not change the vehicle treatment, but the HOCOS treatment did significantly from 5 weeks of treatment. Furthermore, at 7 weeks of treatment, animals treated with vincristine followed by treatment with HOCOS showed no significant differences compared to animals that did not receive vincristine.

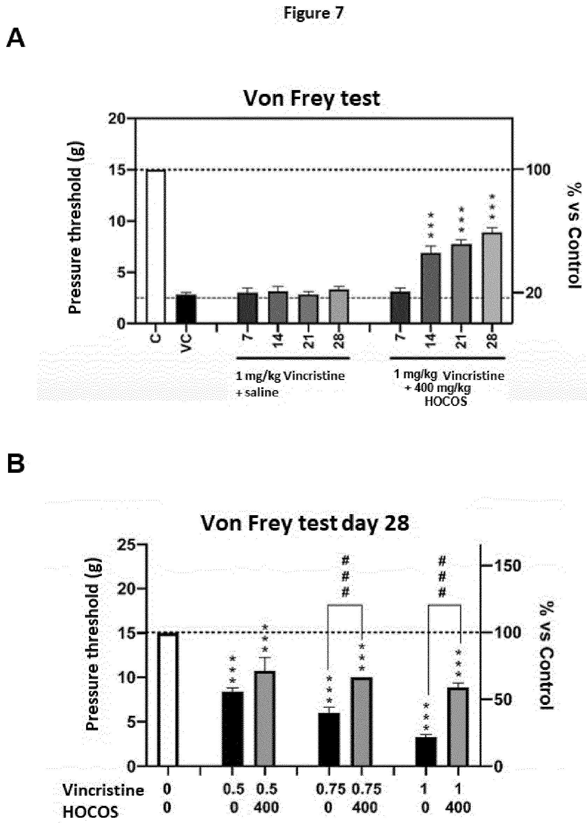

**Figure 7. Efficacy of HOCOS measured on mechanical allodynia by means of the "Von Frey" test against vincristine-induced neuropathic pain.** To identify the effect of HOCOS against other types of neuropathic pain other than those caused by spinal cord injury, the effect was studied using the Von Frey test. **(A)** The threshold pressure (grams) that induces the plantar reflex in animals previously treated with vincristine (VC, cumulative dose of 1 mg/kg in 10 days) and after 1, 14, 21 and 28 days of treatment in the absence (0 mg/kg) or presence of HOCOS (400 mg/g). **(B)** Result of animals previously treated with 0 (control), 0.5, 0.75 or 1 mg/kg vincristine and subsequent treatment in the absence (0 mg/kg) or presence of HOCOS (400 mg/kg) for 28 days. The results were expressed as both grams of pressure and % of pressure with respect to the control.

## DESCRIPTION OF EMBODIMENTS

### Example 1: Phase I/II clinical trial in patients with glioblastoma and other advanced cancers

An open-label multicentre phase I/II trial (without placebo) was conducted in patients with glioblastomas (high-grade with native or mutated IDH) and high-grade gliomas and other advanced solid tumours in continuous growth phase. All enrolled patients gave their written informed consent and were also receiving the concomitant medication listed in the present invention. The trial was conducted in accordance with The Declaration of Helsinki and The International Council on Harmonization Guidelines on Good Clinical Practice and was approved by the Institutional Review Board or Ethics Committees of participating institutions. The trial was designed by Laminar Pharmaceuticals S.A., in collaboration with the trial investigators.

The primary objectives were to determine the safety and tolerability **of HOCOS administered as monotherapy,** and to describe the maximum tolerated dose (MTD), dose limiting toxicity (DLT) and identify the recommended dose for treatment. Secondary objectives included characterizing the unique and steady-state pharmacokinetic profiles of HOCOS on a continuous daily dosing schedule, and evaluating the preliminary antitumour efficacy of HOCOS.

The disease was documented by computed tomography or magnetic resonance imaging. Tumors were evaluated at baseline and every two treatment cycles until disease progression according to the RANO criteria for glioblastomas, gliomas, and other nervous system tumours, or RECIST 1.1 for solid tumours in other tissues and organs.

We included patients older than 18 years with histologically or cytologically confirmed advanced solid tumours, including glioblastomas, and various types of high-grade gliomas refractory to standard treatment, or for which there is no standard treatment (Table 2) who had previously been treated with more than one (up to five) different line of cancer treatment and no longer responded to conventional treatments. In the glioma cohort, patients with grade 3 or 4 malignant gliomas that had relapsed or progressed after standard first or second line treatment were included, with progressive disease being defined according to the RANO criteria.

Key exclusion criteria included having received anticancer therapy within four weeks prior to the start of treatment (six weeks for mitomycin and nitrosureas and two weeks for palliative radiotherapy and surgery); NCI-CTCAE grade > 1 unresolved from prior anticancer therapy; gastrointestinal dysfunction that could alter drug absorption; history of hyperlipidemia and/or need for lipid-lowering therapy; significant uncontrolled cardiovascular disease; and recent intracranial or intratumoral bleeding on computed tomography or magnetic resonance imaging.

HOCOS was administered in the form of a dry powder and accompanied by a series of excipients (Table 1), in envelopes of polyester, aluminium and low-density polystyrene, and was reconstituted as an oral suspension in water between 30 minutes and 2 hours after a meal in 21-day cycles. The suspension contained the amount of HOCOS and the excipients indicated in Table 1 for the different dose levels.

**Table 1. HOCOS formulations for the treatment of cancer**

| Dose HOCOS<br>Compound | 0 g<br>(placebo) | 0.125 g | 0.25 g | 0.5 g | 1 g | 2 g | 3 g | 4 g |
|---|---|---|---|---|---|---|---|---|
| HOCOS | 0.000 | 0.125 | 0.250 | 0.500 | 1.000 | 2.000 | 3.000 | 4.000 |
| Microcrystalline cellulose | 4.0 | 3.875 | 3.750 | 3.500 | 3.000 | 2.000 | 1.000 | |
| Mannitol | 3.680 | 3.680 | 3.680 | 3.680 | 3.680 | 3.680 | 3.680 | 3.680 |
| Citric Acid | 0.011 | 0.015 | 0.030 | 0.060 | 0.115 | 0.225 | 0.330 | 0.350 |
| Sucralose | 0.050 | 0.050 | 0.050 | 0.050 | 0.050 | 0.050 | 0.050 | 0.050 |
| Xanthan gum | 0.100 | 0.100 | 0.100 | 0.100 | 0.100 | 0.100 | 0.100 | 0.100 |
| Mango Flavour | 0.300 | 0.300 | 0.300 | 0.300 | 0.300 | 0.300 | 0.300 | 0.300 |
| **Total weight** | **8.141** | **8.145** | **8.16** | **8.19** | **8.245** | **8.355** | **8.46** | **8.48** |
| **Water for Reconstitution** | **100 ml** | **100 ml** | **100 ml** | **100 ml** | **100 ml** | **100 ml** | **100 ml** | **100 ml** |

The initial dose was 250 mg twice a day (b.i.d.), that is, 500 mg daily. A standard "3+3" dose escalation design was used with seven cohorts (total daily dose of 500 mg to 16000 mg, see Table 2). The doses of 500, 1000, 2000, 4000, 8000 and 16000 mg daily were obtained by applying 2 daily administrations (b.i.d.), while the dose of 12000 mg daily was obtained by administering 4000 mg three times a day (t.i.d.). Three patients per dose level were enrolled and observed for any dose limiting toxicity (DLT) during the first treatment cycle. They were moved to higher dose levels if no DLT was observed in any patient. An additional 3 patients were included at a given dose level if 1 in 3 patients experienced DLT. As a standard safety measure, within each cohort, patients were admitted with an interval greater than or equal to 1 week between the first patient and the following ones.

To be evaluable for dose escalation decisions, patients must have taken more than 80% of the trial drug and not have a DLT during the DLT observation period.

Cohort dose escalation decisions were also based on a clinical review of all available relevant data from contemporary and previous dose cohorts. The maximum dose administered was defined as the dose level at which a DLT was observed during the treatment cycle in more than 33% of the evaluable patients, and the maximum tolerated dose (MTD) was defined as the highest dose level below the previous one.

Treatment was maintained until clinical or radiological disease progression, unacceptable toxicity, withdrawal of consent, or investigator decision. Modifications of the treatment schedule, dose delays of up to 14 days and up to 2 dose reductions due to toxicity were allowed. Dose increases were allowed within the patient if the patient had obtained a clinical benefit with their initial or current dose level, at the discretion of the investigator and in consultation with the medical monitor.

Pharmacokinetics were assessed by MS tandem HPLC to measure HOCOS concentrations. Pharmacokinetic profiles (pre-dose, and 1, 2, 4, 6 and 8 hours post-dose) were measured in cycle 1, days 1 and 21. This included blood pharmacokinetic exposure (dose, concentration, maximum concentration [Cmax], area under the time-plasma curve [AUC]) and population pharmacokinetic parameters (volume of distribution [Vd] and clearance [CL]). In the dose escalation cohorts, pre-dose trough levels were also measured on days 8 and 15. In the extended safety cohort, only the pharmacokinetic profile of day 1 was measured. A final pharmacokinetic sample was taken at the final trial visit.

The predicted maximum sample size was determined by modelling the dose escalation clinical stop criteria, with additional patients in the 2 extended safety cohorts.

The efficacy analysis population included all patients who received at least an amount equal to or greater than 80% of the doses administered in cycle 1 and who underwent at least one tumour evaluation in the trial. Responses were summarized descriptively using frequency distributions. The median progression-free survival (PFS) was estimated by Kaplan-Meier analysis. Progression-free survival at 6 months (PFS, i.e. the percentage of patients alive and progression-free at 180 days after the start of treatment) was also determined.

The pharmacokinetic analysis population included patients who received HOCOS and provided at least one blood sample for evaluable pre-dose and post-dose pharmacokinetics. Pharmacokinetic parameters were summarized by descriptive statistics. Individual and mean concentration versus time profiles were presented on a linear and logarithmic scale. A power model was used to test dose proportionality.

## Results

54 patients were treated in 5 research centres in the United Kingdom and Spain (Table 2). No patients were withdrawn due to an adverse event (AE) or a serious adverse event (SAE) in both the dose escalation phase and the expansion phase of the trial. Thirty-two patients (15 with glioblastoma/glioma and 17 with other advanced solid tumours) were treated in the first 7 cohorts and included in the dose escalation phase (500 mg/day to 16000 mg/day) of the trial. Next, 22 patients (12 with glioma and 10 with advanced solid tumours) were included in the expansion cohort (cohort 8) and all patients received the trial drug at a dose of 12000 mg/day. Of these patients, 44 were evaluable to analyse the efficacy of HOCOS, since to evaluate this parameter the patients had to have undergone at least two radiological analyses.

The mean cumulative dose was 318571.4 mg (1143-2912571 mg), and the mean duration of treatment was 41 days (2-989). Dose escalation was from 500 to 16000 mg/day. HOCOS was rapidly absorbed, with dose-proportional exposure (Figure 1). Promising activity was observed in patients with high-grade gliomas. The clinical trial was highly positive, since none of the 21 patients with glioblastoma and high-grade gliomas showed serious adverse effects and the average PFS was 40 days, with responses of up to 3 years. The PFS at 18 months in these patients with a life expectancy of a few weeks was a hopeful 18.5%. On the other hand, patients with other solid tumours in different organs had a PFS of 42 days on average. In conclusion, HOCOS demonstrated a very good safety profile and an encouraging preliminary activity in this population of patients with tumours in the brain and other organs, of a malignant nature, difficult to treat and with a very low life expectancy. It should be noted that these patients are in continuous tumour progression and have already stopped responding to any type of therapy, so both the arrest of disease progression (stable disease, SD) and partial responses (tumour reductions: *partial response,* PR) are considered positive responses to therapy.

In the group of patients with glioblastomas and gliomas, a decrease in the brain-specific protein of astroglia cells *(glial fibrillary acidic* protein, GFAP) was observed in 12 of 15 patients (80%) on day 8 of cycle 1 (4 hours post-dose), with a median percentage change from baseline of - 20.1%. In this context, GFAP levels correlate with tumour volume and constitute a potential glioma biomarker. The trend was less marked in cycle 2, on day 1 (pre-dose), with a decrease in GFAP observed in 8 of 13 (61.5%) patients and a median percent change from baseline of -10.51%.

Twenty-one (21) patients with glioblastoma or glioma and other neurological tumours (Table 2) underwent radiological evaluation (*MRI*) at baseline and at at least one time point after baseline. In this patient population, both partial responses (PR) and stable disease (SD) were observed according to the RANO criteria (Figure 2A, Table 2). One patient experienced a sustained partial response of more than 3 years with 1000 mg HOCOS daily between cycle 1 and cycle 44, and then with 12000 mg daily between cycle 45 and cycle 48 (Figure 2A and Figure 3A). All patients had received 2 lines of treatment without bevacizumab. The mean SD was 40 days, with 18.5% of patients having a disease-free period of 6 months or more.

In addition, another 24 patients with other advanced solid tumours were included in the trial in the population planned for treatment. In these patients, reductions in tumour size were also observed in some and disease stabilization in others (Figure 2B, Table 2), with the mean tumour stabilization being 42 days.

In this first phase I/IIA human trial, HOCOS monotherapy was well tolerated, and the most frequent adverse effects were diarrhoea, nausea, and vomiting, which occurred infrequently and could be managed with medication. The events were dose-related and predictable given the health status of the patient population, as well as the nature of the HOCOS oral suspension formulation. There were no AEs attributable to the trial drug or AEs that resulted in death. Therefore, HOCOS was well tolerated at the dose of 4000 mg three times a day (12 grams p.o., t.i.d.).

HOCOS monotherapy demonstrated promising antitumour activity in patients with high-grade glioma (grade 3) and glioblastoma (grade 4) and with other cancers. It could be observed that patients with glioblastoma, glioma and other types of cancer experienced PR or SD according to the RANO criteria, with a clinical benefit that lasted more than 6 months in 5 patients, including a patient with grade IV glioblastoma who had been pretreated, but no longer responded to any treatment and who experienced a response that lasted more than 3 years (Figure 3A). The progression-free survival (PFS) rate at 6 months in the glioma population was 18.5%, which is surprising for this type of patients, who no longer respond to any treatment when their disease is in progression and their death usually occurs within a few months of recurrence.

The encouraging clinical response observed in a population with such a poor prognosis and with clear unmet medical needs, supports the therapeutic efficacy of this product.

**Table 2. Patients treated in the clinical trial with HOCOS monotherapy**

| Patient code | Dose (g/day) | Pathology | No. of cycles | No. of previous treatments |
|---|---|---|---|---|
| 010101 | 0.5 | Mesothelioma | 2 | 1 |
| 020102 | 0.5 | Mesothelioma | 15 | 2 |
| 010103 | 0.5 | Glioblastoma | 2 | 2 |
| 030201 | 1 | Glioblastoma | 1 | 4 |
| 010202 | 1 | Glioblastoma | 49 | 2 |
| 030203 | 1 | Glioblastoma | | 4 |
| 020204 | 1 | Adenocarcinoma Pancreas | 2 | 2 |
| 030301 | 2 | Glioblastoma | 2 | 2 |
| 010302 | 2 | Glioblastoma | 2 | 1 |
| 020303 | 2 | Small cell lung cancer (SCLC) | 2 | 4 |
| 030401 | 2 | Endometrial adenocarcinoma | 2 | 5 |
| 020402 | 4 | Rectal adenocarcinoma | 2 | 8 |
| 010403 | 4 | Rectal metastatic adenocarcinoma | 1 | 3 |
| 010404 | 4 | Glioma | 1 | 1 |
| 010501 | 8 | Colon adenocarcinoma | 2 2 | 5 |
| 030502 | 8 | Urachal adenocarcinoma | 2 | 12 |
| 020503 | 8 | Metastatic sigmoid adenocarcinoma of the colon | 2 | 9 |
| 010601 | 12 | Metastatic rectal cancer | 3 | 6 |
| 030602 | 12 | Glioblastoma | 1 | 4 |
| 030603 | 12 | Glioblastoma | 9 | 2 |
| 030604 | 12 | Colon adenocarcinoma | 1 | 11 |
| 030605 | 12 | Oligoastrocytoma | 1 | 2 |
| 010606 | 12 | Glioblastoma | 9 | 2 |
| 030607 | 12 | Astrocytoma | 3 | 3 |
| 020608 | 12 | Adenocarcinoma of the recto-sigmoid junction | 2 | 12 |
| 010701 | 16 | Glioma G3 | 1 | 2 |
| 030702 | 16 | Astrocytoma | 2 | 2 |
| 020703 | 16 | Pleural mesothelioma | 1 | 4 |
| 010704 | 16 | Uterine chondrosarcoma | 1 | 4 |
| 030705 | 16 | Endometrial adenocarcinoma | 2 | 7 |
| 020706 | 16 | Metastatic adenocarcinoma of the lung | 2 | 3 |
| 030707 | 16 | Anaplastic astrocytoma | 3 | 4 |
| 013001 | 12 | Glioblastoma | 2 | 3 |
| 033002 | 12 | Glioblastoma | 2 | 1 |
| 053003 | 12 | Glioma | 1 | 1 |
| 013004 | 12 | Oligodendroglioma | 14 | 2 |
| 033005 | 12 | Glioblastoma | 1 | 4 |
| 013006 | 12 | Glioblastoma | 10 | 2 |
| 033007 | 12 | Glioblastoma | 2 | 6 |
| 033008 | 12 | Gliosarcoma | 3 | 9 |
| 013009 | 12 | Gliomatosis cerebris | 2 | 2 |
| 023010 | 12 | Glioblastoma | 1 | 2 |
| 033011 | 12 | Glioblastoma | 1 | 5 |
| 013012 | 12 | Glioblastoma | 2 | 1 |
| 024001 | 12 | Metastatic colorectal adenocarcinoma | 2 | 4 |
| 034002 | 12 | Colon adenocarcinoma | 4 | 7 |
| 034003 | 12 | Rectal adenocarcinoma | 2 | 8 |
| 014004 | 12 | Colorectal Carcinoma | 2 | 6 |
| 044005 | 12 | Distal bile duct adenocarcinoma | 7 | 3 |

(continued)

| Patient code | Dose (g/day) | Pathology | No. of cycles | No. of previous treatments |
|---|---|---|---|---|
| 024006 | 12 | Metastatic small cell esophageal cancer | 1 | 3 |
| 014007 | 12 | Metastatic rectal adenocarcinoma | 3 | 6 |
| 034008 | 12 | Rectal cancer | 3 | 14 |
| 044009 | 12 | Bladder adenocarcinoma | 2 | 4 |
| 014011 | 12 | Adenocarcinoma of the caecum | 2 | 5 |

HOCOS monotherapy, prescribed as rescue treatment in cancer patients, who have been previously treated with other agents, showed an encouraging antitumour activity, together with a manageable safety profile, considerably less toxic than other chemotherapies; which places it as an ideal candidate for the first-line treatment of gliomas, grade IV glioblastomas with native trial, and other types of cancer.

HOCOS treatment has not only been evidenced in humans, but also in animal models of human cancers of various types, such as pancreatic cancer. Figure 3B shows the antitumour effect of HOCOS on the growth of human pancreatic cancer (of MIAPaCa-2 cells) in immunocompromised mice. In this case, pancreatic cancer is a very aggressive type of cancer, with a high mortality rate and a very short life expectancy. In this way, patients with distant pancreatic cancer have a 5-year survival of 3%. This survival is somewhat higher in patients who have regional pancreatic cancer (16%), with localized cancer having the best prognosis (44% life expectancy at 5 years).

**Inclusion criteria**

Patients with glioblastoma/glioma and other types of tumours were included in this phase I/II clinical trial. These patients were administered HOCOS monotherapy with the doses indicated in Table 2. The criteria for including these patients were as follows:

- Able and willing to give written informed consent.
- Male or female patients $\geq$ 18 years of age.
- With a histologically or cytologically confirmed advanced solid neoplasm that is refractory to standard treatment or for which there is no standard therapy.

**Inclusion criteria for patients with neurological tumours**

- Have a diagnosis of glioblastoma, grade 3 or 4 malignant glioma, or similar neurological tumours, that recur (relapse) or progress after standard first- or second-line treatment or subsequent lines.
- Truly progressive disease, confirmed according to the RANO criteria.
- A life expectancy of at least 12 weeks.
- ECOG performance status of 0-2.
- Able to swallow and ingest oral medication.
- Ability to undergo an adequate tumour image, by CT or MRI, to evaluate the evolution of the disease.
- Hematologic values at screening/baseline: haemoglobin $\geq$ 90 g/L (9 g/dL) or 5.6 mmol/L, absolute neutrophil count $\geq$ 1.5 x $10^9$/L, platelets $\geq$ 100 x $10^9$/L.
- Clotting values at screening/baseline: International Normalized Ratio (INR) $\leq$ 1.5, partial thromboplastin time (PTT) $\leq$ 2 x upper limit of normal (ULN).
- Liver function test values at screening/baseline: total bilirubin $\leq$ 1.5 $\times$ ULN - unless explained by a genetic syndrome such as Gilberts syndrome; alanine aminotransferase (ALT) and aspartate aminotransferase (AST) $\leq$ 2.5 $\times$ ULN.
- Renal function test value at screening/baseline: creatinine serum $\leq$ 1.5 x ULN.
- No history of corrected QT interval prolongation (QTc), and a normal QTc interval at the time of screening/baseline (QTc $\leq$ 450 msec).
- Female patients (or male patient whose partner is) with no potential to procreate (defined as > 2 years after the last menstruation or surgically sterile), female patients with potential to procreate with a negative serum pregnancy test within 7 days prior to the first dose of HOCOS or within 14 days, followed by a negative confirmatory urine pregnancy test within 7 days prior to the first dose of HOCOS, and using (or if they are male and not surgically sterile, whose partner is using) effective and non-hormonal contraceptive means (non-hormonal intrauterine contraceptive device, barrier contraceptive method together with spermicidal gel).

**Inclusion criteria for patients with solid tumours other than glioblastoma/glioma**

- The presence of lesions suitable for biopsy (mandatory for non-glioma patients enrolled in the extended safety cohort and highly desirable for non-glioma patients enrolled in the dose escalation phase).

**Example 2: First-line clinical trial in newly diagnosed glioblastoma patients with HOCOS in combination with temozolomide and radiotherapy**

Following the good results of the phase I/II clinical trial, a phase II/III trial was conducted in patients newly diagnosed with glioblastoma. This clinical trial was carried out in a double-blind, placebo-controlled format. 27 patients were included in this. Of the total patients, 50% were randomly assigned to the placebo group and the other half were assigned to the HOCOS group, without the doctor or the patient knowing who was taking placebo or *verum*. Two of the recruited patients withdrew from the trial before starting the treatment, so they were not valid for the analysis. Patients with newly diagnosed glioblastoma or grade IV glioma and with the native isocitrate dehydrogenase (IDH) enzyme (without mutations) and methylation of the MGMT gene promoter were included in the trial. All patients received concomitantly, in addition to temozolomide, other drugs mentioned in the present invention. In addition, patients received chemotherapy following the protocol indicated above.

The treatment used in the clinical trial consisted of 5 phases:

Step 1. Treatment of the patient with surgery for the removal of glioblastoma.
Step 2. Submit the patient to chemoradiotherapy lasting between 6 to 7 weeks comprising the administration of radiotherapy in daily fractions of 2 Gy, 5 days per week; temozolomide in a dose of 75 mg/m$^2$ per day administered concomitantly with radiotherapy; HOCOS in doses of 12,000 mg/day or placebo.
Step 3. A 4-week washout period where HOCOS and temozolomide radiotherapy is discontinued.
Step 4. A maintenance period of 6 cycles, where each cycle has a duration of 4 weeks and each cycle consists of administering temozolomide (150-200 mg/m$^2$) and HOCOS (12000 mg/day) or a placebo daily for the first three weeks of each cycle and then 7 days of rest without treatment for each cycle.
Step 5. Maintenance monotherapy, 4-week cycles consisting of administering HOCOS (12000 mg/day) or placebo daily for the first 3 weeks of each cycle, followed by 7 days of washout without treatment, where said cycles continue permanently as maintenance therapy until the tumour shows progression, according to RANO criteria.

The clinical trial conducted was a randomized, double-blind, placebo-controlled, 2-parallel-arm (1:1 ratio) study to evaluate the efficacy and safety of HOCO sodium salt versus placebo in patients newly diagnosed with glioblastoma, (grade IV glioma) and with native trial. In all arms, patients received the standard treatment (temozolomide and radiotherapy) together with different drugs cited in the present invention and were randomized to receive placebo (arm A) or a dose of 12000 mg/day of HOCOS (arm B).

This trial evaluates progression-free survival (PFS), as the period of time between when glioblastoma is diagnosed and when tumour enlargement is observed based on RANO criteria. In addition, the life time from diagnosis, called overall survival (OS), is studied.

**Results**

Survival (both OS and PFS) of 24 patients has been monitored over a period of 36 months. Figure 4 shows the results of this trial. The 1:1 randomization (1 *placebo-treated* patient for each HOCOS-treated patient) indicates that half of the patients received an investigational product (HOCOS) and the other half received placebo. Figure 4A shows the overall survival (OS) of the patients participating in the trial carried out in the present invention (black bars: combinatorial HOCOS + temozolomide), compared to the survival of the standard treatment (SoC, grey bars: temozolomide only). As can be seen, the overall survival of patients in the mixed population treated with temozolomide plus HOCOS or placebo is much higher (60%, 44% and 36% survival at 17, 28 and 36 months from diagnosis, respectively) than that of standard treatment with temozolomide as the only chemotherapy (38%, 9.4% and 1.4% survival at 17, 28 and 36 months, after diagnosis, respectively). A similar increase is observed in progression-free survival (Figure 4B), given that the percentages of PFS at 17, 28 and 36 months were 28%, 24% and 8%, respectively, while with standard treatment the PFS were 18%, 2.8% and 0.35%, respectively. These data clearly indicate that patients treated with HOCOS plus temozolomide derive additional therapeutic benefit from treatment with temozolomide as the sole chemotherapy agent. Considering that approximately half of the patients are treated with HOCOS and the other half are treated with placebo, the potential percentage of patients benefited by this new chemical compound was calculated as:

$$0.5 \times (\% \text{ HOCOS}) + 0.5 \times (\% \text{ SoC}) = \% \text{ MIN}$$

Where % MIN is the percentage of patients with survival (PFS or OS, as the case may be) in the mixed population of patients treated and not treated with HOCOS of the trial of the present invention, % SoC is the percentage of patients that appear in the studies carried out with the standard treatment (temozolomide only) and % HOCOS is the percentage of patients with survival (PFS or OS, as the case may be) that would be if all patients with grade IV glioblastoma multiforme with native IDH were treated with HOCOS (plus temozolomide and the drugs cited in the present invention). In this equation, % HOCOS is the only unknown. % MIN data are obtained from the present trial and % SoC data were obtained from the literature (meta-analysis with all trials conducted in patients with glioblastoma and treated with temozolomide). This calculation, which indicates the therapeutic potential of HOCOS, clearly demonstrates the therapeutic benefit of this compound, since the survival rates, i.e. OS and PFS values, at 36 months were 70.6% and 15.7% for HOCOS + temozolomide and only 1.4% and 0.35% for temozolomide alone (Figure 4). These results indicate that HOCOS produces a marked and significant increase in the life expectancy of patients with native IDH grade IV glioblastoma, and that its combined use with temozolomide can save many lives. In addition, its oral administration and absence of adverse effects mean that HOCOS can be administered permanently as the only antitumour maintenance therapy, while temozolomide can only be administered for 6 months due to its toxicity.

**Example 3. Assays in cell lines with HOCOS in combination with other compounds in the treatment of various types of cancer**

In addition to the monotherapy treatments, both in humans and in human cancer cells and experimental animals shown in Figure 2A, Figure 2B and in Figure 3A and Figure 3B, the combinatorial treatments between HOCOS and other compounds extend beyond what is shown in Figure 4. This is possible for 2 reasons. The first is that HOCOS is a very harmless (non-toxic) compound and there is no risk of increasing the significant and dangerous adverse effects that other anti-tumour drugs have. The second is that the mechanism of action of HOCOS is totally different from that of other drugs and, therefore, its effects can be additive. To study the possible use of HOCOS in combinatorial therapies, human glioblastoma cells of lines U118, SNB19, SF268, human mesothelioma cells NCI-H2021 and pancreatic cancer cells BxPC-3 (pancreatic adenocarcinoma) and MIAPaCa-2 (neuroendocrine pancreatic cancer) were cultured in the absence or presence of different chemotherapeutic agents and combinations of HOCOS with said compounds. The compounds used in this trial were temozolomide, thiamine, gemcitabine, fluoroacyl, oxyplatin, irinotecan, etoposide, imatinib, folfirinox, erlotinib, and cisplatin. The results obtained show that the combination of HOCOS with temozolomide is about 3 times more potent than HOCOS alone (Table 3). In addition, it was observed that all the combinations used had an antitumour potency superior to that of a single drug, both on glioblastoma and pancreatic cancer. Therefore, since HOCOS is a compound without toxicity at therapeutic doses and with a mechanism of action different from the rest of antitumors that increases the potency of these, it can be used in combinatorial therapies together with other drugs for the treatment of glioblastoma or other types of cancer.

**Table 3. Efficacy of HOCOS combined with other antitumour drugs**

| Compound | $IC_{50}$ | Combination | $IC_{50}$ | Cell and cancer type |
|---|---|---|---|---|
| Thiamine | 15.35 mM | Thiamine + 200 $\mu$M HOCOS | 5.75 mM | SNB19 Glioblastoma |
| HOCOS | 1119 $\mu$M | HOCOS + 300 $\mu$M Temozolomide | 472 $\mu$M | SF268 Glioblastoma |
| Etoposide | 2.08 $\mu$M | Etoposide + 150 $\mu$M HOCOS | 1.25 $\mu$M | U118 Glioblastoma |
| Irinotecan | 14.96 $\mu$M | Irinotecan + 150 $\mu$M HOCOS | 5.47 $\mu$M | U118 Glioblastoma |
| Imatinib | 234.4 nM | Imatinib + 150 $\mu$M HOCOS | 169.8 nM | U118 Glioblastoma |
| Gemcitabine | 7.8 nM | Gemcitabine + 300 $\mu$M HOCOS | 5 nM | BxPC-3 Pancreas |
| HOCOS | 450.1 $\mu$M | HOCOS + 2 $\mu$M fluoroacyl + 2 $\mu$M oxyplatin + 1 $\mu$M irinotecan | 201.1 $\mu$M | MIAPaCa-2 Pancreas |
| Folfirinox | 1 $\mu$M | HOCOS + Folfirinox | 0.4 $\mu$M | BxPC-3 |
| Erlotinib | 16.03 $\mu$M | Erlotinib + 150 $\mu$M HOCOS | 6.27 $\mu$M | A549 Lung |
| Cisplatin | 16.37 | Cisplatin + 150 HOCOS | 5.47 $\mu$M | NCI-H2021 Mesothelioma |

**Example 4: Clinical trial in patients with neuropathic pain caused by spinal cord injuries**

A clinical trial was conducted with HOCOS in people with spinal cord injury who suffered from neuropathic pain. The objective of the trial was to demonstrate the pharmacological safety (absence of toxicity) and pharmacological efficacy against neuropathic pain in patients with nerve injury. In this clinical trial, the average neuropathic pain of the patients who participated in the research was between 7 and 8, with 10 being considered the maximum pain that can be had and 0 the absence of pain, according to the VAS scale. Therefore, it is a population with elevated chronic pain. A pain that, despite the many analgesic drugs they use, is not mitigated as it is a pathology different from conventional pain.

In a population of 44 patients, patients were randomly segregated (double-blind) into 4 groups. HOCOS tablets were administered at doses of 0 (placebo), 1050, 2100 or 4200 milligrams per day (in 3 doses and by oral administration) and neuropathic pain was recorded according to the VAS scale as a measure of efficacy. In addition, all patients received concomitant medication described in the present invention. The composition of said tablets is described in Table 4.

**Table 4. HOCOS tablet composition for the treatment of neuropathic pain**

| COMPONENTS | AMOUNT PER TABLET (mg) | PLACEBO AMOUNT (mg) |
|---|---|---|
| Active component (AHO) | 350.0 | 0.0 |
| Microcrystalline cellulose | 331.0 | 681.0 |
| Mannitol | 255.0 | 255.0 |
| Hydroxypropylcellulose SSL | 24.0 | 24.0 |
| Croscarmellose sodium | 60.0 | 60.0 |
| Sodium glycolate (starch) | 60.0 | 60.0 |
| Hydroxypropylcellulose LH11 | 60.0 | 60.0 |
| Sodium lauryl sulphate | 12.0 | 12.0 |
| Ascorbic acid | 30.0 | 30.0 |
| Sodium stearyl fumarate | 18.0 | 18.0 |
| Pure water | q.s. | q.s. |
| Total weight of the base tablet | 1200 mg | 1200 mg |
| Casing: Opadry® con II 88A18002 White Polyvinyl Alcohol-Part. Hydrolysated (E-120) Talcum (E-553b) Titanium dioxide (E-171) Fatty acid-glycerol esters Sodium lauryl sulphate | 48 mg | 48 mg |
| Pure water | q.s. | q.s. |
| Total weight of tablet casing | 1248 mg | 1248 mg |

To select those patients who met the inclusion requirements for the trial, mainly having spinal cord injury, having neuropathic pain and receiving pregabalin treatment, the patients underwent a screening visit (SV). Once selected for the trial, a first visit (V1) was made in which they were informed of the trial, pain was quantified, they were administered the necessary medication for one month and a blood sample was taken to evaluate the patient's baseline status. After 1, 2 and 3 months of treatment, visits V2, V3 and V4, respectively, were performed to evaluate the effectiveness of the treatment and take blood samples. From V4, the medication was discontinued and a visit was performed after one month without treatment (V5). Therefore, the V5 was a visit in which an increase in the level of pain was expected in all patients, both in those in whom the treatment worked and stopped taking the product, and in those patients who had a placebo effect. At all visits, screening and V1 to V5, pain was measured with the VAS scale (Table 5).

**Table 5. Neuropathic pain data (VAS scale) in patients with spinal cord injury**

| HOCOS dose (g/day) | Patient code | SV | V1 | V2 | V3 | V4 (EoT) | V5 (EoS/FU) | SV-V4 | V1-V4 | V5-V4 |
|---|---|---|---|---|---|---|---|---|---|---|
| 0[a] | HPT012 | 5 | 6.7 | 4.5 | 3.2 | 4 | 1 | 1 | 2.7 | -3 |
| 0 | HPT018 | 6.6 | 5.7 | 5.5 | 8 | 7 | 7.5 | -0.4 | -1.3 | 0.5 |
| 0 | HTP025 | 6.7 | 5.8 | 5.1 | 5.5 | 5 | 5.4 | 1.7 | 0.8 | 0.4 |

(continued)

| HOCOS dose (g/day) | Patient code | SV | V1 | V2 | V3 | V4 (EoT) | V5 (EoS/FU) | SV-V4 | V1-V4 | V5-V4 |
|---|---|---|---|---|---|---|---|---|---|---|
| 0 | HIG029 | 9 | 9 | 5 | 3 | 2 | 1 | 7 | 7 | -1 |
| 0 | HPT034 | 8.8 | 8.4 | 8 | 7.5 | 7 | 5.5 | 1.8 | 1.4 | -1.5 |
| 0 | HPT037 | 9.6 | 9.6 | 9.2 | 9 | 9.5 | 9.2 | 0.1 | 0.1 | -0.3 |
| 0 | HUC047 | 6.5 | 6.5 | 7 | 8 | 8 | 7 | -1.5 | -1.5 | -1 |
| 0 | HVH049 | 6.5 | 7 | 7 | 6.5 | 6 | 6.5 | 0.5 | 1 | 0.5 |
| 0 | HPT063 | 6.77 | 6.84 | 5.54 | 2.44 | 1 | 4.3 | 5.77 | 5.84 | 3.3 |
| 1.05 | HPT002 | 5.6 | 6.6 | 6.4 | 6.7 | 6.3 | 6.1 | -0.7 | 0.3 | -0.2 |
| 1.05 | HVH008 | 7.8 | 6.5 | 3 | 3 | 2 | 4 | 5.8 | 4.5 | 2 |
| 1.05 | HPT011 | 7.8 | 4 | 7.3 | 2.5 | 6.7 | 7 | 1.1 | -2.7 | 0.3 |
| 1.05 | HPT017 | 5.2 | 3.5 | 3.8 | 1.4 | 4 | 6.2 | 1.2 | -0.5 | 2.2 |
| 1.05 | HUC021 | 7.05 | 7.5 | 7 | 7.7 | 7.5 | 4 | -0.45 | 0 | -3.5 |
| 1.05 | HPT032 | 8.2 | 8.2 | 7.5 | 7.5 | 6.7 | 7.8 | 1.5 | 1.5 | 1.1 |
| 1.05 | HPT039 | 4.5 | 4.7 | 4.7 | 6.5 | 6.5 | 8 | -2 | -1.8 | 1.5 |
| 1.05 | HPT046 | 8.2 | 8.6 | 8.4 | 7.5 | 7 | 7.5 | 1.2 | 1.6 | 0.5 |
| 1.05 | HLM051 | 8.9 | 8.9 | 6.2 | 4.9 | 7.7 | 9.4 | 1.2 | 1.2 | 1.7 |
| 1.05 | HIG056 | 7 | 7 | 5 | 4 | 5 | 5 | 2 | 2 | 0 |
| 1.05 | HPT058 | 6 | 5 | 4.7 | 5.5 | 4.4 | 4.5 | 1.6 | 0.6 | 0.1 |
| 1.05 | HPT061 | 10 | 9 | 8 | 6.73 | 7.2 | 8 | 2.8 | 1.8 | 0.8 |
| 2.1 | HPT001 | 6.5 | 6.5 | 8.1 | 7.1 | 7.35 | 7.3 | -0.85 | -0.85 | -0.05 |
| 2.1 | HVH005 | 6.4 | 5 | 4 | 3 | 2.5 | 8 | 3.9 | 2.5 | 5.5 |
| 2.1 | HIG024 | 7.5 | 7.5 | 7.5 | 6 | 6 | 6 | 1.5 | 1.5 | 0 |
| 2.1 | HPT031 | 4 | 5 | 2.5 | 1.4 | 2.6 | 2 | 1.4 | 2.4 | -0.6 |
| 2.1 | HIG033 | 8 | 8 | 1 | 2 | 2 | 1.5 | 6 | 6 | -0.5 |
| 2.1 | HIG035 | 7 | 7 | 7 | 7.5 | 5 | 6 | 2 | 2 | 1 |
| 2.1 | HLM042 | 7.7 | 7.7 | 6.5 | 4 | 2.7 | 8.2 | 5 | 5 | 5.5 |
| 2.1 | HPT052 | 5.2 | 4.5 | 6.5 | 3.5 | 4 | 4 | 1.2 | 0.5 | 0 |
| 2.1 | HPT060 | 6.66 | 6.67 | 7.52 | 5.43 | 5.37 | 8.49 | 1.29 | 1.3 | 3.12 |
| 2.1 | HPT062 | 10 | 10 | 6.2 | 5.7 | 8.3 | MD | 1.7 | 1.7 | |
| 2.1 | HPT066 | 3.9 | 5.1 | 6 | 3.7 | 1.7 | 3.1 | 2.2 | 3.4 | 1.4 |
| 4.2 | HPT007 | 5 | 5 | 4 | 4.5 | 5 | MD | 0 | 0 | |
| 4.2 | HVN014 | 6.5 | 6.5 | 4.5 | 4.5 | 5 | 6.5 | 1.5 | 1.5 | 1.5 |
| 4.2 | HPT022 | 8.3 | 8.4 | 8.4 | 8 | 8.8 | 8.8 | -0.5 | -0.4 | 0 |
| 4.2 | HVH027 | 6 | 6 | 6 | 6 | 6 | 6 | 0 | 0 | 0 |
| 4.2 | HLM041 | 6.2 | 6.2 | 4.4 | 6 | 5.7 | 5.2 | 0.5 | 0.5 | -0.5 |
| 4.2 | HUC044 | 7 | 7 | 6 | 4 | 1.7 | 4.5 | 5.3 | 5.3 | 2.8 |
| 4.2 | HUC045 | 8.5 | 10 | 7 | 7.2 | 5 | 9 | 3.5 | 5 | 4 |
| 4.2 | HVN050 | 8.7 | 9 | 9 | 8.7 | 6.6 | 7.8 | 2.1 | 2.4 | 1.2 |
| 4.2 | HVR053 | 8.5 | 7.6 | 7.8 | 5.2 | 5.4 | 7 | 3.1 | 2.2 | 1.6 |
| 4.2 | HPT059 | 4.4 | 5.7 | 3 | 3.7 | 6.7 | 6.7 | -2.3 | -1 | 0 |
| 4.2 | HIG064 | 5 | 5 | 7 | 5 | 9 | 3 | -4 | -4 | -6 |
| 4.2 | HVR065 | 7.4 | 8.1 | 8.1 | 8 | 7.2 | 8.9 | 0.2 | 0.9 | 1.7 |

**Measurement of adverse effects of HOCOS**

In this clinical trial, HOCOS produced fewer adverse effects per patient (P w/AE) than placebo: 1 adverse effect per placebo patient versus 0.5 adverse effects per HOCOS-treated patient (Figure 5A). Moreover, treatment-attributable adverse events were also higher in patients receiving placebo (0.44 adverse events per patient, placebo treatment-related, P w/RTAE) than in patients receiving HOCOS (0.08 adverse events per patient, HOCOS treatment-related, P w/RTAE) at the optimal dose (2100 milligrams per day). The reduction of more than 80% in this parameter in the group with the dose selected for subsequent treatments (2100 milligrams daily) suggests that HOCOS is safe (non-toxic) and that, in addition, it protects against general health problems and/or problems derived from another medication mentioned in this invention in patients with spinal cord injury. Thus, these data indicate that HOCOS has a positive effect on the overall health of patients with spinal cord injury (paraplegia and quadriplegia).

**Measurement of the efficacy of HOCOS against neuropathic pain**

The efficacy of HOCOS against neuropathic pain in patients with spinal cord injury was determined using the VAS scale which consists of a straight line whose ends define "no pain" (0 on the scale) and the "worst possible pain" (10 on the scale) (Haefeli and Elfering). The patient is asked to mark the intensity of their pain between these two points. Length scales between 5 and 20 cm have been used, although the best results have been obtained with lengths of 10 to 15 cm. In this clinical trial, the efficacy of HOCOS was evaluated in 44 patients with spinal cord injury and neuropathic pain using 2 baseline measures, the screening visit (SV) and the first visit (V1), in which the patient had not yet received medication. Oral treatment lasted 3 months and pain was assessed after one month (V2), two months (V3) and 3 months (V4) of treatment. V4 was also called an End of Treatment (EoT) visit. Patients remained medication-free for one month and were subsequently evaluated at a fifth visit (V5), also called an End of Study (EoS) visit.

The analysis of efficacy was performed both in the "Total Population" of the trial (44 patients: Figure 5B), as in a "Censored Population" of patients with objectively permanent pain (34 patients in whom when treatment ceased, a withdrawal effect to HOCOS was observed with increased pain: Figure 5C). The parameter selected to censor the patients was the pain behaviour between visits 4 and 5. Since the clinical trial shown in the present invention lasted about 6 months from patient screening to EoS, it is possible that several patients entered a cycle of pain reduction spontaneously and naturally. Patients who have shown a reduction in pain due to treatment experience an increase in pain after the end of the treatment period, so those patients whose pain decreased at the end of treatment, between V4 and V5, were censored. This censorship was carried out equivalently in all experimental groups. Additionally, pain was analysed in the group of **responding censored patients,** those who had a reduction in pain greater than 1 point on the VAS scale (Figure 5D). As can be seen, there is a significant reduction in patients treated with HOCOS, especially in the 2.1 gram daily group (700 milligrams 3 times a day, Tables 4 and 5). This difference is significant when censored patients, whose pain does not rise between V4 and V5, are eliminated. There are no large differences in absolute values of reduction between the 3 analyses performed with (1) all patients (Figure 5B), (2) uncensored patients (Figure 5C), and (3) responding censored patients (Figure 5D), indicating that overall pain decrease is not affected by this type of analysis. The statistical significance, however, does improve between these analyses, which shows that more homogeneous populations of patients are studied.

As discussed above, neuropathic pain differs from other types of algesic pain and is caused by different molecular and cellular mechanisms. The PDQ questionnaire allows us to determine if the pain suffered by the patient is neuropathic or not. In a further *post hoc* analysis, it could be observed that patients receiving treatments of 2100 mg/day and 4200 mg/day had significant reductions in neuropathic pain levels measured by the VAS (Figure 5E). In this case, the dose of 2100 mg/day (p.o., t.i.d) was the most effective, with the decrease in pain being evident from the first month of treatment and significant from the second month. After 3 months of treatment with 2100 mg/day, the VAS values in these patients with a high probability of suffering from neuropathic pain were approximately half with respect to the start of treatment (Figure 5E). In this subpopulation of patients, in addition, it could be observed that the percentage of responders in the placebo group (0 mg/day) was only 25% (Figure 5F). In contrast, 80% of patients receiving 2100 mg/day of HOCOS responded to treatment, and those receiving 1050 mg/day and 4200 mg/day responded 37% and 55%, respectively (Figure 5F).

These results indicate that HOCOS is effective in treating neuropathic pain. It is very important to consider that all patients in this trial were treated with pregabalin and other medications, which only achieved partial pain relief. The reduction observed in patients due to treatment with HOCOS is important (up to 3 points on the VAS scale), and also occurs in addition to the effect of pregabalin and the other medications. Since HOCOS has a different mechanism of action than other compounds, it can induce this effect both alone and in combination with other drugs. In fact, the patients in this trial received different combinations of drugs in each case, so the efficacy is not exclusively related to any of the combinations. In other words, HOCOS may present efficacy as monotherapy or combinatorial therapy with pregabalin, opioids, anticonvulsants, anxiolytics, anesthetics, antidepressants, etc.

In the uncensored population of 2100 milligrams daily (most effective therapeutic dose), it showed its efficacy in 75% of patients, in which the average effect was approximately 2 points of reduction in the VAS pain scale (Tables 5 and 6). In a previous trial with pregabalin (Cardenas et al.) it was observed that only approximately 50% of patients responded to treatment with a mean reduction of 1.2 points on the VAS scale. Therefore, it can be concluded that HOCOS is more effective than pregabalin and, in addition, that its effect overlaps that of pregabalin, being additive to it and to the rest of the medications mentioned in the present invention.

The additive effect of HOCOS, together with the rest of the drugs, to induce a marked reduction in neuropathic pain has several considerations of interest that must be highlighted. Firstly, this reduction is greater than that produced by the reference drug for the treatment of neuropathic pain, pregabalin (together with the other drugs mentioned here), which only decreases neuropathic pain by 1.2 points on the VAS scale (Cardenas et al.). On the other hand, the percentage of patients responding to pregabalin was 33% in the placebo group and 48% in the pregabalin group, with a difference of 0.82 points between these groups on the VAS scale (Cardenas et al.). In this trial with a daily dose of 2.1 g of HOCOS, there were pain reductions of 1.92 points on the VAS scale between V1 and V4 for the total population. Patient censorship did not result in differences in absolute pain values, but in the significance of the trial according to the statistical test used (two-way ANOVA, Fisher's LSD test). Regarding the percentage of patients responding to HOCOS treatment in the censored population, decreases greater than 1 VAS point were observed in 55% of patients treated with 1050 milligrams per day, 75% in those treated with 2100 mg/day and 56% in those treated with 4200 mg/day (Table 6). In contrast, only 17% of placebo-treated and censored patients showed marked reductions in neuropathic pain (Table 6). These data clearly indicate that HOCOS is more effective than pregabalin. Moreover, all patients in this trial were on pregabalin treatment, so the effect observed is additive to that of pregabalin. This additive effect is due to the difference in mechanism of action of both compounds. In the subpopulation of patients with high probability of having neuropathic pain, the effect of the 2100 mg/day dose of HOCOS at 3 months was very marked and significant (Figure 5E). Moreover, at 4200 mg/day, significant differences in pain reduction were also found, while placebo or low doses (1050 mg/day of HOCOS) did not produce significant changes in pain values on the VAS scale in patients with neuropathic pain. This demonstrates that treatment with HOCOS produces a specific pharmacological effect on neuropathic pain in patients with spinal cord injury. Moreover, after the end of the treatment, the increase in mean value on the VAS scale is also consistent with a specific effect of this compound. Finally, in the patient population with a high probability of having neuropathic pain (> 90%) according to the PDQ, the percentage of treatment responders in the 2100 mg/day group is markedly higher than in the placebo group (25% and 80%, respectively: Figure 5F). Again, this result demonstrates that treatment with HOCOS is specific and concentration-dependent.

**Table 6. Total patients, uncensored and responders**

| Dose (g/day) | Patients evaluated | Uncensored Patients [1] | Responder patients [2] | % of uncensored responders |
|---|---|---|---|---|
| 0 | 9 | 6 | 1 | 17 |
| 1.05 | 12 | 11 | 6 | 55 |
| 2.1 | 11 | 8 | 6 | 75 |
| 4.2 | 12 | 9 | 5 | 56 |

[1] patients who experienced no placebo or HOCOS withdrawal effect between V4 and V5 were censored for the second analysis.
[2] patients with marked reduction in pain (V4-V1>1 VAS analysis).

**Example 5. Efficacy of HOCOS against neuropathic pain in a vincristine-treated rat model**

The efficacy of HOCOS against neuropathic pain was also investigated in a rat model treated with vincristine (0.5 mg/kg to 1 mg /kg), a drug used in cancer chemotherapy and associated with the onset of neuropathic pain. This model induces peripheral neuropathy similar to that which could be observed in other types of patients, such as those with fibromyalgia, chemotherapy pain, post-herpetic pain or after diabetes or hepatitis, since it does not occur due to traumatic nerve injury. Therefore, this model is different from that of spinal cord injury and is an example of the usefulness of HOCOS in other types of neuropathic pain other than nerve injuries, in general, and spinal cord injury, in particular. In experimental animals, the level of pain was recorded using analgesimetres based on the reduction of hyperalgesia (exacerbated pain produced by mild stimuli, such as pressure with a toothpick) or allodynia (pain produced by non-painful, such as gentle pressures, the rubbing of a sheet or a finger on the skin). In this sense, 2 types of tests were carried out. (1) The "tail flick" test allows the measurement of thermal hyperalgesia and consists of applying a heat source on the tail, by means of a laser light beam or similar, and recording the time it takes the animal to move its tail away from the light beam. The longer the animal endures, the higher the temperature it resists and the lower its pain threshold. This test measures the latency or period (seconds) between the start of the stimulus in the animal and its response to it, so that lower latency values correspond to a higher level of neuropathic pain (Klazas et al.). In this sense, while vincristine dose-dependently reduced the latency time, HOCOS increased said time in treated animals, indicating that it is able to reverse the neuropathic pain induced by vincristine (Figure 6A). In addition to the dose-dependent effect of vincristine, treatment with HOCOS after treatment with vincristine (1 mg/kg) had effects dependent on the time of treatment (Figure 6B). In this way, HOCOS, but not the vehicle, induced recoveries in tail flick values. These results demonstrate that HOCOS is very effective for treating neuropathic pain caused by causes other than nerve injuries. Likewise, these results demonstrate the possibility of using HOCOS in the treatment of cancer patients and patients who present pain induced by antitumour chemotherapeutic agents, such as various alkaloids, including vincristine.

In addition to the measures of thermal hyperalgesia, mechanical allodynia was measured, using the Von Frey test. In this test, a non-painful stimulus (for example, a plantar pressure with a plastic fibre) is induced and the animal's reaction to said stimulus is observed. Figure 5 shows how the effect of HOCOS, measured by the Von Frey test, is very marked and significant, at the same time it increases during the treatment time (0-28 days) and is maintained against high doses of cytotoxic agent (Figures 7A and 7B). Mechanical allodynia is a type of pain that frequently appears in patients suffering from fibromyalgia, chemotherapy pain, post-herpetic pain or after diabetes or hepatitis. Therefore, HOCOS is useful to treat these types of neuropathic pain.

The above tests and results demonstrate that HOCOS is effective for the treatment of neuropathic pain originating from causes other than nerve or spinal cord injuries.

**REFERENCES**

Brandsma and van den Bent. Pseudoprogression and pseudoresponse in the treatment of gliomas. Curr Opin Neurol. 2009;22(6):633-8. doi: 10.1097/WCO.0b013e328332363e

Cardenas et al. A randomized trial of pregabalin in patients with neuropathic pain due to spinal cord injury. Neurology. 2013;80(6):533-9. doi: 10.1212/WNL.0b013e318281546b

Haefeli and Elfering. Pain assessment. Eur Spine J. 2006;15 Suppl 1(Suppl 1):S17-24. doi: 10.1007/s00586-005-1044-x

Klazas et al. Gabapentin Increases Intra-Epidermal and Peptidergic Nerve Fibers Density and Alleviates Allodynia and Thermal Hyperalgesia in a Mouse Model of Acute Taxol-Induced Peripheral Neuropathy. Biomedicines. 2022;10(12):3190. doi: 10.3390/biomedicines10123190.

Lencioni et al. Objective response by mRECIST as a predictor and potential surrogate endpoint of overall survival in advanced HCC. J Hepatol. 2017;66(6):1166-1172. doi: 10.1016/j.jhep.2017.01.012.

Rodgers et al., Morphine resistance in spinal cord injury-related neuropathic pain in rats is associated with alterations in dopamine and dopamine-related metabolomics. J Pain. 2022;23(5):772-783. doi: 10.1016/j.jpain.2021.11.009.

**Claims**

1. The compound 2-hydroxy-octadecene-9-cis-oate, or a pharmaceutically acceptable salt or ester thereof, for use in the treatment of an oncological pathology selected from the group consisting of glioblastoma, astrocytoma, pilocytic astrocytoma, diffuse astrocytoma, anaplastic astrocytoma, oligoastrocytoma, oligodengroglioma, ependymoma, xanthastrocytoma, medulloblastoma, low-grade glioma, grade 3 glioma, pontine glioma, ependymal tumour, subependymoma, gliomatosis cerebri, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, choroid plexus tumour, choroid plexus papilloma, pineocytoma, pineoblastoma, mesothelioma, pleural mesothelioma, bile duct adenocarcinoma, exocrine pancreatic cancer, neuroendocrine pancreatic cancer, metastatic lung adenocarcinoma, small cell lung cancer, colon adenocarcinoma, rectal cancer, recto-sigmoid junction cancer, rectal adenocarcinoma, metastatic rectal adenocarcinoma, metastatic sigmoid adenocarcinoma of the colon, colorectal adenocarcinoma, metastatic colorectal adenocarcinoma, urachal fistula cancer, urachal adenocarcinoma, endometrial adenocarcinoma, neuroendocrine pancreatic cancer, pancreatic adenocarcinoma, uterine chondrosarcoma, blind bowel adenocarcinoma, bladder adenocarcinoma, esophageal cancer and metastatic small cell esophageal cancer, which is administered at a dose of 500 mg/day to 16000 mg/day.

2. The compound, salt or ester for use according to claim 1, wherein the oncological pathology is selected from the group consisting of oligoastrocytoma, glioblastoma, oligodengroglioma, ependymoma, xanthoastrocytoma, medulloblastoma, pilocytic astrocytoma, pontine glioma, ependymal tumour, subependymoma, gliomatosis cerebri, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, tumour of the choroid plexus, papilloma of the choroid plexus, pineocytoma, pineoblastoma, mesothelioma, pleural mesothelioma, bile duct adenocarcinoma, exocrine pancreatic cancer, neuroendocrine pancreatic cancer, metastatic lung adenocarcinoma, small cell lung cancer, rectal cancer, recto-sigmoid junction cancer, metastatic rectal adenocarcinoma, metastatic sigmoid adenocarcinoma of the colon, colorectal metastatic adenocarcinoma, urachal fistula cancer, urachal adenocarcinoma, endometrial cancer, neuroendocrine pancreatic cancer, uterine chondrosarcoma, blind bowel adenocarcinoma, bladder adenocarcinoma, esophageal cancer, and metastatic small cell esophageal cancer.

3. The compound, salt or ester for use according to claim 1 or 2, wherein the glioblastoma is grade IV glioblastoma multiforme with the native isocitrate dehydrogenase (IDH) enzyme.

4. The compound, salt or ester for use according to claim 3, for use in the treatment of native IDH grade IV glioblastoma multiforme in a subject having methylation of the promoter of the methyl guanine methyl transferase (MGMT) gene.

5. The compound, salt or ester for use according to any one of claims 1 to 4, wherein the salt is sodium salt.

6. The compound, salt or ester for use according to any one of claims 1 to 5, wherein the ester is methyl ester or ethyl ester.

7. The compound, salt or ester for use according to any one of claims 1 to 6, wherein the compound, salt or ester is administered orally.

8. The compound, salt or ester for use according to any one of claims 1 to 7, which is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day, 8000 mg/day, 10000 mg/day, 12000 mg/day, 12600 mg/day, 14000 mg/day and 16000 mg/day.

9. The compound, salt or ester for use according to any one of claims 1 to 8, which is administered in a dose of 2100 mg/day.

10. The compound, salt or ester for use according to any one of claims 1 to 8, which is administered in a dose of 12000 mg/day.

11. The compound, salt or ester for use according to any one of claims 1 to 10, wherein the compound, salt or ester is used as a first-line treatment.

12. The compound, salt or ester for use according to claim 11, wherein the first-line treatment comprises 4-week cycles, wherein each cycle comprises administering the compound, salt or ester daily for the first three weeks of each cycle and wherein during the fourth week no treatment is administered.

13. The compound, salt or ester for use according to any one of claims 1 to 12, wherein the compound, salt or ester is used as a pre-surgical or post-surgical treatment to decrease the size of a tumour.

14. The compound, salt or ester for use according to any one of claims 1 to 12, wherein the subject receiving the treatment has been previously treated with at least one and up to five different chemotherapeutic treatment lines.

15. The compound, salt or ester for use according to any one of claims 1 to 10, 13 or 14, wherein the compound, salt or ester is used as a maintenance treatment.

16. The compound, salt or ester for use according to claim 15, wherein the maintenance treatment comprises 4-week cycles, wherein each cycle comprises administering the compound, salt or ester daily for the first three weeks of each cycle and wherein during the fourth week no treatment is administered.

17. The compound, salt or ester for use according to any one of claims 1 to 16, wherein the treatment comprises:

- a chemoradiotherapy phase lasting from 6 to 7 weeks, comprising administering radiotherapy daily, 5 days per week; administering a second chemotherapeutic agent in a daily dose of 75 mg/m$^2$ from the first day of radiotherapy; and administering the compound, salt or ester in a dose of 12000 mg/day for 4 weeks from week 3 counted from the start of radiotherapy;

- a 4-week treatment rest period;
- a 4-week, 6-cycle maintenance period, wherein each cycle comprises administering the second chemotherapeutic agent at a daily dose of 150-200 mg/m$^2$, the first five days of each cycle; administering the compound, salt or ester at a dose of 12000 mg/day for the first three weeks of each cycle and wherein during the fourth week no treatment is administered; and
- a monotherapy phase of 4-week cycles, wherein each cycle comprises administering the compound, salt or ester in a dose of 12000 mg/day during the first three weeks of each cycle, wherein during the fourth week no treatment is administered, wherein said cycles continue indefinitely as maintenance therapy.

18. The compound, salt or ester for use according to any one of claims 1 to 17, for simultaneous, separate or sequential use in combination with a second chemotherapeutic agent selected from the group consisting of temozolomide, thiamine, gemcitabine, fluorouracil, oxyplatin, irinotecan, etoposide, imatinib, folfirinox, erlotinib and cisplatin.

19. The compound, salt or ester for use according to claim 18, for simultaneous, separate or sequential use in combination with temozolomide, in the treatment of glioblastoma.

20. The compound, salt or ester for use according to claim 19, wherein the glioblastoma is native IDH grade IV glioblastoma multiforme.

21. The compound 2-hydroxy-octadecene-9-cis-oate, or a pharmaceutically acceptable salt or ester thereof, for use in the treatment of neuropathic pain, which is administered at a dose of 500 mg/day to 16000 mg/day.

22. The compound, salt or ester for use according to claim 21, wherein the neuropathic pain is caused by a neuronal injury or by a nerve injury of the central and/or peripheral nervous system.

23. The compound, salt or ester for use according to claim 22, wherein the neuronal injury is a spinal cord injury.

24. The compound, salt or ester for use according to claim 21, wherein the neuropathic pain is caused by a cause selected from the group consisting of a chemotherapeutic agent, an antitumour agent and cancer.

25. The compound, salt or ester for use according to claim 24, wherein the antitumour agent is an alkaloid.

26. The compound, salt or ester for use according to claim 25, wherein the alkaloid is vincristine.

27. The compound, salt or ester for use according to claim 21, wherein the neuropathic pain is caused by a cause selected from the group consisting of peripheral diabetic neuropathy, post-herpetic neuralgia, fibromyalgia and hepatitis.

28. The compound, salt or ester for use according to any one of claims 21 to 27, which is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day, 8000 mg/day, 10000 mg/day, 12000 mg/day, 12600 mg/day, 14000 mg/day and 16000 mg/day.

29. The compound, salt or ester for use according to any one of claims 21 to 28, which is administered in a dose of 2100 mg/day.

30. The compound, salt or ester for use according to any one of claims 21 to 28, which is administered in a dose of 4200 mg/day.

31. The compound, salt or ester for use according to any one of claims 21 to 30, for simultaneous, separate or sequential use in combination with at least one other active ingredient selected from the group consisting of pregabalin, an opioid analgesic, a steroidal analgesic, a non-steroidal analgesic, a cannabinoid analgesic, an anticonvulsant, an anxiolytic, an anaesthetic and an antidepressant.

32. The compound, salt or ester for use according to any one of claims 21 to 31, for simultaneous use, separately or sequentially in combination with at least one other active ingredient selected from the group consisting of lamotrigine, omeprazole, phenoxypentanoic acid, metformin, ibuprofen, dulcolax, alprazolam, diazepam, baclofen, macrogol, duloxetine, levofloxacin, gabapentin, ceftriaxone, enoxaparin, pantoprazole, paracetamol, ipratropium bromide, acetylcysteine, metoclopramide, metamizole, teicoplanin, dexketoprofen, oxybutynin, meropenem, beclomethasone dipropionate, formoterol fumarate, atorvastatin, calcifediol, paroxetine, cyanocobalamin, clonazepam, amlodipine, trazodone, flurazepam, thiazides, losartan, metamizole, sucralfate, lactitol, pancreatin, dimethicone, bethanechol, amitriptyline, lorazepam, vitamin D, lormetazepam, solifenacin, simvastatin, cinitapride, quetiapine, betmiga, valproate, lacosamide, lidocaine, metamizole, finasteride, acetylsalicylic acid, enalapril, metformin, liraglutide, trospium, tramadol, celcoxib, citalopram, sildenafil, tamsulosin, loratadine, tizanidine, vortioxetine, zolpidem, bromazepam, dexamethasone, dexketoprofen, losartan, eslicarbazepine, pentoxifylline, mirabegron, fluoxetine, clorazepate, mirtazapine, rosuvastatin, prednisone, beclamethasone, methylprednisone, acyclovir, lignocaine, nystatin, delorazepam, alendronic acid, carbamazepine, cortisone, hydrocortisone, cannabidiol and tetrahydrocannabinol.

33. A pharmaceutical composition comprising the compound 2-hydroxy-octadecene-9-cis-oate, or a pharmaceutically acceptable salt or ester thereof, together with at least one pharmaceutically acceptable excipient or carrier, for use in the treatment of an oncological pathology selected from the group consisting of glioblastoma multiforme, astrocytoma, pilocytic astrocytoma, diffuse astrocytoma, anaplastic astrocytoma, oligoastrocytoma, oligodengroglioma, ependymoma, xanthastrocytoma, medulloblastoma, low-grade glioma, grade 3 glioma, pontine glioma, ependymal tumour, subependymoma, gliomatosis cerebri, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, choroid plexus tumour, choroid plexus papilloma, pineocytoma, pineoblastoma, mesothelioma, pleural mesothelioma, bile duct adenocarcinoma, exocrine pancreatic cancer, neuroendocrine pancreatic cancer, metastatic lung adenocarcinoma, small cell lung cancer, colon adenocarcinoma, rectal cancer, recto-sigmoid junction cancer, rectal adenocarcinoma, metastatic rectal adenocarcinoma, metastatic sigmoid adenocarcinoma of the colon, colorectal adenocarcinoma, metastatic colorectal adenocarcinoma, urachal fistula cancer, urachal adenocarcinoma, endometrial adenocarcinoma, neuroendocrine pancreatic cancer, pancreatic adenocarcinoma, uterine chondrosarcoma, blind bowel adenocarcinoma, bladder adenocarcinoma, esophageal cancer and metastatic small cell esophageal cancer, wherein the compound, salt or ester is administered in a dose of 500 mg/day to 16000 mg/day.

34. The pharmaceutical composition according to claim 33, wherein the oncological pathology is selected from the group consisting of oligoastrocytoma, glioblastoma, oligodengroglioma, ependymoma, xanthoastrocytoma, medulloblastoma, pilocytic astrocytoma, pontine glioma, ependymal tumour, subependymoma, gliomatosis cerebri, embryonic tumour, atypical rhabdoid teratoid tumour, tumour of cranial and spinal nerves, mixed neuro-glial tumour, pituitary tumour, germ cell tumour, meninges tumour, meningioma, hemangiopericytoma, hemangioblastoma, tumour of the choroid plexus, papilloma of the choroid plexus, pineocytoma, pineoblastoma, mesothelioma, pleural mesothelioma, bile duct adenocarcinoma, exocrine pancreatic cancer, neuroendocrine pancreatic cancer, metastatic adenocarcinoma of the lung, small cell lung cancer, rectal cancer, recto-sigmoid junction cancer, metastatic rectal adenocarcinoma, metastatic sigmoid adenocarcinoma of the colon, metastatic colorectal adenocarcinoma, urachal fistula cancer, urachal adenocarcinoma, endometrial cancer, neuroendocrine pancreatic cancer, uterine chondrosarcoma, blind bowel adenocarcinoma, bladder adenocarcinoma, esophageal cancer and metastatic small cell esophageal cancer.

35. The pharmaceutical composition according to claim 33 or 34, wherein the glioblastoma is grade IV glioblastoma multiforme with the native isocitrate dehydrogenase (IDH) enzyme.

36. The pharmaceutical composition according to claim 35, for use in the treatment of native IDH grade IV glioblastoma multiforme in a subject having methylation of the promoter of the methyl guanine methyl transferase (MGMT) gene.

37. The pharmaceutical composition according to any one of claims 33 to 36, wherein the salt is sodium salt.

38. The pharmaceutical composition according to any one of claims 33 to 37, wherein the ester is methyl ester or ethyl ester.

39. The pharmaceutical composition according to any one of claims 33 to 38, wherein the compound, salt or ester is administered in a dose selected from the group consisting of 500 mg/day, 1000 mg/day, 1050 mg/day, 2000 mg/day, 2100 mg/day, 3150 mg/day, 4000 mg/day, 4200 mg/day, 6000 mg/day, 6300 mg/day, 8000 mg/day, 10000 mg/day, 12000 mg/day, 12600 mg/day, 14000 mg/day and 16000 mg/day.

40. The pharmaceutical composition according to any one of claims 33 to 39, wherein the compound, salt or ester is administered in a dose of 2100 mg/day.

41. The pharmaceutical composition according to any one of claims 33 to 39, wherein the compound, salt or ester is administered in a dose of 12000 mg/day.

42. The pharmaceutical composition according to any one of claims 33 to 41, further comprising a second chemotherapeutic agent selected from the group consisting of temozolomide, thiamine, gemcitabine, fluorouracil, oxyplatin, irinotecan, etoposide, imatinib, folfirinox, erlotinib, and cisplatin.

43. The pharmaceutical composition according to any one of claims 33 to 42, further comprising temozolomide, for use in the treatment of glioblastoma.

44. The pharmaceutical composition according to claim 43, wherein the glioblastoma is native IDH grade IV glioblastoma multiforme.

45. A pharmaceutical composition comprising the compound 2-hydroxy-octadecene-9-cis-oate, or a pharmaceutically acceptable salt or ester thereof, together with at least one pharmaceutically acceptable excipient or carrier, for use in the treatment of neuropathic pain, wherein the compound, salt or ester is administered in a dose from 500 mg/day to 16000 mg/day.

46. The pharmaceutical composition according to claim 45, wherein the neuropathic pain is caused by a neuronal injury or by a nerve injury of the central and/or peripheral nervous system.

47. The pharmaceutical composition according to claim 46, wherein the neuronal injury is a spinal cord injury.

48. The pharmaceutical composition according to claim 45, wherein the neuropathic pain is caused by a cause selected from the group consisting of a chemotherapeutic agent, an antitumour agent and cancer.

49. The pharmaceutical composition according to claim 48, wherein the antitumour agent is an alkaloid.

50. The pharmaceutical composition according to claim 49, wherein the alkaloid is vincristine.

51. The pharmaceutical composition according to claim 45, wherein the neuropathic pain is caused by a cause selected from the group consisting of diabetic peripheral neuropathy, postherpetic neuralgia, fibromyalgia and hepatitis.

52. The pharmaceutical composition according to any one of claims 45 to 51, wherein the compound, salt or ester is administered in a dose selected from the group consisting of 500 mg/day, 1000 mg/day, 1050 mg/day, 2000 mg/day, 2100 mg/day, 4000 mg/day, 4200 mg/day, 6000 mg/day, 8000 mg/day, 10000 mg/day, 12000 mg/day, 14000 mg/day and 16000 mg/day.

53. The pharmaceutical composition according to any one of claims 45 to 52, wherein the compound, salt or ester is administered in a dose of 2100 mg/day.

54. The pharmaceutical composition according to any one of claims 45 to 52, wherein the compound, salt or ester is administered in a dose of 4200 mg/day.

55. The pharmaceutical composition according to any one of claims 45 to 54, further comprising at least one other active ingredient selected from the group consisting of pregabalin, an opioid analgesic, a steroidal analgesic, a non-steroidal analgesic, a cannabinoid analgesic, an anticonvulsant, an anxiolytic, an anesthetic, and an antidepressant.

56. The pharmaceutical composition according to claim 55, further comprising at least one other active ingredient selected from the group consisting of lamotrigine, omeprazole, phenoxypentanoic acid, metformin, ibuprofen, dulcolax, alprazolam, diazepam, baclofen, macrogol, duloxetine, levofloxacin, gabapentin, ceftriaxone, enoxaparin, pantoprazole, paracetamol, ipratropium bromide, acetylcysteine, metoclopramide, metamizole, teicoplanin, dexketoprofen, oxybutynin, meropenem, beclomethasone dipropionate, formoterol fumarate, atorvastatin, calcifediol, paroxetine, cyanocobalamin, clonazepam, amlodipine, trazodone, flurazepam, thiazides, losartan, metamizole, sucralfate, lactitol, pancreatin, dimethicone, bethanechol, amitriptyline, lorazepam, vitamin D, lormetazepam, solifenacin, simvastatin, cinitapride, quetiapine, betmiga, valproate, lacosamide, lidocaine, metamizole, finasteride, acetylsalicylic acid, enalapril, metformin, liraglutide, trospium, tramadol, celcoxib, citalopram, sildenafil, tamsulosin, loratadine, tizanidine, vortioxetine, zolpidem, bromazepam, dexamethasone, dexketoprofen, losartan, eslicarbazepine, pentoxifylline, mirabegron, fluoxetine, clorazepate, mirtazapine, rosuvastatin, prednisone, beclamethasone, methylprednisone, acyclovir, lignocaine, nystatin, delorazepam, alendronic acid, carbamazepine, cortisone, hydro-cortisone, cannabidiol and tetrahydrocannabinol.

# Figure 1

# Figure 2

Figure 3

A

B

# Figure 4

**A**

**B**

**Figure 5**

# Figure 5 (Cont.)

# Figure 5 (Cont.)

## Figure 6

**A**

**B**

## Figure 7

**A**

**B**

## INTERNATIONAL SEARCH REPORT

| | International application No. |
|---|---|
| | PCT/ES2024/070157 |

| A. CLASSIFICATION OF SUBJECT MATTER |
|---|

See extra sheet

According to International Patent Classification (IPC) or to both national classification and IPC

| B. FIELDS SEARCHED |
|---|

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, TXT-DB, XPESP,NPL, BIOSIS,CAS

| C. DOCUMENTS CONSIDERED TO BE RELEVANT |
|---|

| Category* | Citation of document, with indication, where appropriate, of the relevant passages | Relevant to claim No. |
|---|---|---|
| P,X | LOPEZ JUANITA et al. "A Phase 1/2A trial of idroxioleic acid: first-in-class sphingolipid regulator and glioma cell autophagy inducer with antitumor activity in refractory glioma". British Journal Of Cancer Sep 21 2023, Vol. 129, N° 5, pages 811-818, ISSN 0007-0920(print) ISSN 1532-1827(electronic), <DOI: 10.1038/s41416-023-02356-1>. See abstract; results. | 1-56 |
| A | ES 2401629 A1 (UNIV ILLES BALEARS) 23/04/2013, pages 9, 10, 15 and 30; example 6; figure 8; table 2; claims 1-7 and 16. | 1-56 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 29/04/2024 | **(06/05/2024)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | N. Martín Laso Telephone No. 913493278 |

Form PCT/ISA/210 (second sheet) (July 2022)

## INTERNATIONAL SEARCH REPORT

| International application No. |
| --- |
| PCT/ES2024/070157 |

C (continuation).          DOCUMENTS CONSIDERED TO BE RELEVANT

| Category * | Citation of documents, with indication, where appropriate, of the relevant passages | Relevant to claim No. |
| --- | --- | --- |
| A | FERNANDEZ-GARCIA PAULA et al. "The Opposing Contribution of SMS1 and SMS2 to Glioma Progression and Their Value in the Therapeutic Response to 2OHOA". Cancers Jan 2019, 31/12/2018, Vol.11, Nº 1, pages Article No.: 88, ISSN 2072-6694, <DOI: 10.3390/cancers11010088>. See abstract; results. | 1-56 |
| A | WO 2021152201 A2 (UNIV ILLES BALEARS) 05/08/2021, pages 5 and 43-47; example 7; figures 10-14. | 1-56 |
| A | AVILA MARTIN G et al. "Oral 2-hydroxyoleic acid inhibits reflex hypersensitivity and open-field-induced anxiety after spared nerve injury". EUROPEAN JOURNAL OF PAIN, 20140513 SAUNDERS, LONDON, GB., 13/05/2014, Vol. 19, Páginas 111 - 122, ISSN 1090-3801, <DOI: doi:10.1002/ejp.528>. See abstract; results. | 1-56 |
| A | GONZALEZ-ESCALADA, J. R. "Pregabalina in the tratamiento del dolor neuropático periférico". Revista Of The Sociedad Spanish Del Dolor, 2005, Vol. 12, pages 169-180. See abstract. | 1-56 |

Form PCT/ISA/210 (continuation of second sheet) (July 2022)

| Patent document cited in the search report | Publication date | Patent family member(s) | Publication date |
|---|---|---|---|
| ES2401629 A1 | 23.04.2013 | ES2773784T T3 | 14.07.2020 |
| | | RU2017141446 A | 13.02.2019 |
| | | RU2017141446 A3 | 13.02.2019 |
| | | HUE035430T T2 | 02.05.2018 |
| | | PL2774910T T3 | 30.03.2018 |
| | | PT2774910T T | 03.01.2018 |
| | | ES2653675T T3 | 08.02.2018 |
| | | DK2774910T T3 | 08.01.2018 |
| | | EP3287437 A1 | 28.02.2018 |
| | | EP3287437 B1 | 11.12.2019 |
| | | RU2014118123 A | 20.11.2015 |
| | | RU2637937 C2 | 08.12.2017 |
| | | IN3111CHENP2014 A | 03.07.2015 |
| | | CN104321300 A | 28.01.2015 |
| | | JP2014530806 A | 20.11.2014 |
| | | US2014288176 A1 | 25.09.2014 |
| | | US9359281 B2 | 07.06.2016 |
| | | EP2774910 A1 | 10.09.2014 |
| | | EP2774910 A4 | 25.02.2015 |
| | | WO2013050644 A1 | 11.04.2013 |
| WO2021152201 A2 | 05.08.2021 | CA3233141 A1 | 05.08.2021 |
| | | AU2023248158 A1 | 02.11.2023 |
| | | MX2022009035 A | 11.08.2022 |
| | | JP2023518640 A | 08.05.2023 |
| | | CL2022001985 A1 | 03.02.2023 |
| | | US2023097753 A1 | 30.03.2023 |
| | | CA3166307 A1 | 05.08.2021 |
| | | BR112022015172 A2 | 11.10.2022 |
| | | KR20220132552 A | 30.09.2022 |
| | | AU2021212330 A1 | 22.09.2022 |
| | | CN115052852 A | 13.09.2022 |
| | | IL295120 A | 01.09.2022 |
| | | ES2911474 A1 | 19.05.2022 |
| | | ES2911474 B2 | 06.02.2023 |
| | | EP4098649 A2 | 07.12.2022 |
| | | EP4098649 A4 | 17.04.2024 |
| | | ES2846824 A1 | 29.07.2021 |
| | | ES2846824 B2 | 19.01.2022 |

Form PCT/ISA/210 (patent family annex) (July 2022)

## INTERNATIONAL SEARCH REPORT

International application No.

PCT/ES2024/070157

CLASSIFICATION OF SUBJECT MATTER

*A61K31/201* (2006.01)
*A61P35/00* (2006.01)
*A61P25/04* (2006.01)

# EP 4 663 187 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BRANDSMA** ; **VAN DEN BENT**. Pseudoprogression and pseudoresponse in the treatment of gliomas. *Curr Opin Neurol*, 2009, vol. 22 (6), 633-8
- **CARDENAS et al.** A randomized trial of pregabalin in patients with neuropathic pain due to spinal cord injury. *Neurology*, 2013, vol. 80 (6), 533-9
- **HAEFELI** ; **ELFERING**. Pain assessment. *Eur Spine J*, 2006, S17-24
- **KLAZAS et al.** Gabapentin Increases Intra-Epidermal and Peptidergic Nerve Fibers Density and Alleviates Allodynia and Thermal Hyperalgesia in a Mouse Model of Acute Taxol-Induced Peripheral Neuropathy. *Biomedicines*, 2022, vol. 10 (12), 3190

- **LENCIONI et al.** Objective response by mRECIST as a predictor and potential surrogate endpoint of overall survival in advanced HCC. *J Hepatol*, 2017, vol. 66 (6), 1166-1172
- **RODGERS et al.** Morphine resistance in spinal cord injury-related neuropathic pain in rats is associated with alterations in dopamine and dopamine-related metabolomics. *J Pain*, 2022, vol. 23 (5), 772-783